# EUROPEAN PATENT APPLICATION

(11) **EP 4 759 338 A1**
(43) Date of publication of application: **17.06.2026**
(21) Application number: 24220167.1
(22) Date of filing: 16.12.2024
(51) Int. Cl.: A61M 1/16, A61M 1/36, A61M 39/22

(54) **EXTRACORPOREAL BLOOD TREATMENT APPARATUS**

(71) Applicant: Gambro Lundia AB, 226 43 Lund (SE)
(72) Inventor: VERNIN, Guillaume, F-69330 Meyzieu (FR)
(74) Representative: PGA S.p.A., Milano, Succursale di Lugano

(57) **Abstract**

An extracorporeal blood treatment apparatus (100) comprises: a treatment unit (200); an extracorporeal blood circuit (300) comprising a blood withdrawal line (301) connected to the treatment unit (200) and connectable to a first access port (A) or a second access port (B) of a patient and a blood return line (302) connected to an outlet (202) of the treatment unit (200) and connectable to the second access port (B) or the first access port (A) of the patient; a switching valve (1) in fluid communication with the blood withdrawal line (301) and the blood return line (302) and with the first access port (A) and the second access port (B), which is switchable between: (i) a first switching condition, wherein the first access port (A) and the second access port (B) are respectively fluidly connected to the blood withdrawal line (301) and the blood return line (302) to trigger a normal mode of operation; (ii) a second switching condition, wherein the first access port (A) and the second access port (B) are respectively fluidly connected to the blood return line (302) and the blood withdrawal line (301) to trigger a reversing mode of operation wherein blood coming from the patient is circulated through the treatment unit (200) according to an opposite/reverse direction and returned to the patient; (iii) a third switching condition, wherein the first access port (A) and the second access port (B) are fluidly connected to each other and the blood withdrawal line (301) and the blood return blood line (302) are fluidly connected to each other to trigger a recirculation mode, wherein the extracorporeal blood circuit (300) is not in fluid communication with the first (A) and the second access port (B).

## Description

### BACKGROUND

The present disclosure relates generally to medical fluid treatments and in particular to extracorporeal blood treatments, such as dialysis fluid treatments.

Due to various causes, a person's renal system can fail. Renal failure produces several physiological derangements. It is no longer possible to balance water and minerals or to excrete daily metabolic load. Toxic end products of metabolism, such as, urea, creatinine, uric acid and others, may accumulate in a patient's blood and tissue.

Reduced kidney function and, above all, kidney failure is treated with dialysis. Dialysis removes waste, toxins and excess water from the body that normal functioning kidneys would otherwise remove. Dialysis treatment for replacement of kidney functions is critical to many people because the treatment is lifesaving.

One type of kidney failure therapy is Hemodialysis ("HD"), which in general uses diffusion to remove waste products from a patient's blood. A diffusive gradient occurs across the semi-permeable dialyzer between the blood and an electrolyte solution called dialysate or dialysis fluid to cause diffusion. HD fluids are typically created by the dialysis machines by mixing concentrates and clean water.

Hemofiltration ("HF") is an alternative renal replacement therapy that relies on a convective transport of toxins from the patient's blood. HF is accomplished by adding substitution or replacement fluid to the extracorporeal blood circuit during treatment. The substitution fluid and the fluid accumulated by the patient in between treatments is ultrafiltered over the course of the HF treatment, providing a convective transport mechanism that is particularly beneficial in removing middle and large molecules.

Hemodiafiltration ("HDF") is a treatment modality that combines convective and diffusive clearances. HDF uses dialysis fluid flowing through a dialyzer, similar to standard hemodialysis, to provide diffusive clearance. In addition, substitution solution is delivered directly to the extracorporeal blood circuit, providing convective clearance. Here, more fluid than the patient's excess fluid is removed from the extracorporeal blood of the patient, causing the increased convective transport of waste products from the patient. The additional fluid removed is replaced via the substitution or replacement fluid.

The apparatus of the present invention may also be used in the context of continuous renal replacement therapies (CRRT) with or without anticoagulation, for example CRRT with or without systemic anticoagulation (e.g., heparin) / with or without regional anticoagulation (e.g., citrate).

It is known that some pieces of prior art describe use of valves in the medical field and, in particular in connection with extracorporeal blood circuits, capable of providing only two different operation conditions.

Document US2023019233 relates to the field of blood processing systems and, in particular a system including a blood treatment machine, such as a dialyzer. Such a document provides various embodiments of rotary valves which are suitable for serving several fluid paths. All the embodiments disclosed and shown allow only two different modes of operation in which the flow of fluids changes.

Document US6918893 relates to a fluid control valve used to control and inject contrast and saline solutions during an intravascular procedure. The fluid control valve can be coupled to an injector, a saline supply, a contrast supply, a waste outlet or reservoir and a catheter. Three lines couple the control valve to the injector and the catheter. Different embodiments of the fluid control valve are disclosed and illustrated, but, conceptually, they operate in the same way, by providing only two different modes of operation.

Document US2007197951 relates to cardiac surgery, and, more specifically, to the heating and cooling of blood or other fluids delivered to a patient during cardiac bypass surgery. Such a document focuses on a motor-drive rotary valve for supplying cold, warm, or blended cold/warm water for regulating water to a heat exchange unit. The valve provides for a quiet operation and better temperature control of the water supplied to the heat exchange unit. A rotary valve unit is supplied. The rotary valve unit is a multi-port valve to assist in regulating the temperature of the heat transfer fluid to the heat transfer unit. The rotary valve unit includes two inlet ports and two outlet ports. Also in this case, the valve allows only two different modes of operation.

Document US4397335 relates to medical systems, and, in particular a device, the uses of which range from the management of critically ill patients in the intensive care units to the monitoring of patients for open heart surgery, diagnostic cardiac catheterization, and research applications. This document focuses on a rotary valve that has an arrangement of passageways in its rotary valve plug and ports in its housing that permit manual manipulation of the valve to switch the medical system from monitoring pulmonary artery pressure to measuring central venous pressure. The system can also be used to measure cardiac output or to obtain a mixed venous blood sample. The rotary valve comprises a cylindrical housing body having two former ports in axial alignment and two other ports in diametrical opposition to the former ports. The rotary valve further comprises a cylindrical valve plug having a portion insertable into the body. The valve plug is formed with: a pair of diametrically extending passageways spaced from each other axially of the plug and arranged to be in registry with different pairs of ports; a pair of generally helical channels; and, a pair of additional passageways each extending substantially axially along the length of the plug. The rotary valve plug is rotatable between different orientations. However, the valve has only two modes of operation, a normal mode of operation (normal orientation of the passageways) and a reverse mode of operation (crossed orientation of the passageways).

Document US3834372 relates to a manifold used with pressure sensitive transducers to measure a system under pressure and with related sampling and flushing components for cardiac surgery, and, more specifically, to the heating and cooling of blood or other fluids delivered to a patient during cardiac bypass surgery.

### SUMMARY

The present disclosure sets forth extracorporeal blood treatment apparatus, used for performing extracorporeal blood treatments (e.g., renal failure treatments or dialysis), such as hemofiltration (HF), hemodialysis ("HD"), hemodiafiltration ("HDF"), hemoperfusion, toxins adsorption, gas exchange therapies as well as the extracorporeal blood treatments performed during continuous renal replacement treatments ("CRRT").

In light of the disclosure set forth herein, and without limiting the disclosure in any way, in a 1st independent aspect, which may be combined with any other aspect or portion thereof described herein, it is provided an extracorporeal blood treatment apparatus (100) which comprises: a treatment unit (200); an extracorporeal blood circuit (300) comprising a blood withdrawal line (301) connected to an inlet (201) of the treatment unit (200) and connectable to a first access port (A) or a second access port (B), opposite to the treatment unit (200), for the passage of blood inwards the extracorporeal blood circuit (300), and a blood return line (302) connected to an outlet (202) of the treatment unit (200) and connectable to the second access port (B) or the first access port (A), opposite to the treatment unit (200), for the passage of blood outwards the extracorporeal blood circuit (300); at least one switching valve (1) in fluid communication with the blood withdrawal line (301) and the blood return line (302) of the extracorporeal blood circuit (300) and in fluid communication with the first access port (A) and the second access port (B), the switching valve (1) being switchable between: (i) a first switching condition, wherein the first access port (A) and the second access port (B) are respectively fluidly connected to the blood withdrawal line (301) of the extracorporeal blood circuit (300) and the blood return line (302) of the extracorporeal blood circuit (300) to trigger a normal mode of operation of the extracorporeal blood circuit (300) wherein blood coming from the first access port (A) flows through the switching valve (1), the withdrawal line (301) of the extracorporeal blood circuit (300), the treatment unit (200), the blood return line (302) of the extracorporeal blood circuit (300), the switching valve (1) again, to arrive at the second access port (B); (ii) a second switching condition, wherein the first access port (A) and the second access port (B) are respectively fluidly connected to the blood return line (302) of the extracorporeal blood circuit (300) and the blood withdrawal line (301) of the extracorporeal blood circuit (300) to trigger a reversing mode of operation of the extracorporeal blood circuit (300) wherein blood coming from the second access port (B) flows through the switching valve (1), the blood withdrawal line (301) of the extracorporeal blood circuit (300), the treatment unit (200), the blood return line (302) of the extracorporeal blood circuit (300), the switching valve (1) again, to arrive to the first access port (A); (iii) a third switching condition, wherein the first access port (A) and the second access port (B) are fluidly connected to each other and the blood withdrawal line (301) and the blood return blood line (302) of the extracorporeal blood circuit (6) are fluidly connected to each other to trigger a recirculation mode of the extracorporeal blood circuit (300), wherein the extracorporeal blood circuit (300) is not in fluid communication with the first (A) and the second access port (B).

In a 1^{st} bis independent aspect, which may be combined with any other previous or following aspects or portions thereof, an extracorporeal blood treatment apparatus (100) comprises: a treatment unit (200); an extracorporeal blood circuit (300) comprising a blood withdrawal line (301) connected to an inlet (201) of the treatment unit (200) and connectable to a first access port (A), opposite to the treatment unit (200), for the passage of blood inwards the extracorporeal blood circuit (300), and a blood return line (302) connected to an outlet (202) of the treatment unit (200) and connectable to a second access port (B), opposite to the treatment unit (200), for the passage of blood outwards the extracorporeal blood circuit (300); at least one switching valve (1) provided with: a first transit port (2) in fluid communication with the first access port (A); a second transit port (3) in fluid communication with the second access port (B); a first circuit port (4) in fluid communication with the blood withdrawal line (301) of the extracorporeal blood circuit (300); a second circuit port (5) in fluid communication with the blood return line (302) of the extracorporeal blood circuit (300); a selective connection body (6) configured to fluidly connect distinct pair of ports (2, 3, 4, 5) of the switching valve (1), the selective connection body (6) being movable between: (i) a first switching condition, wherein the first transit port (2) of the switching valve (1) is fluidly connected to the first circuit port (4) of the first switching valve (1) to put in fluid communication the first access port (A) with the blood withdrawal line (301) of the extracorporeal blood circuit (300), and the second transit port (3) of the switching valve (1) is fluidly connected to the second circuit port (5) of the switching valve (1) to put in fluid communication the second access port (B) with the blood return line (302) of the extracorporeal blood circuit (300), the first condition triggering a normal mode of operation of the extracorporeal blood circuit (300) wherein blood coming from the first access port (A), flows through the first transit port (2) of the switching valve (1), the selective connection body (6) of the switching valve (1), the first circuit port (4) of the switching valve (1), the withdrawal line (301) of the extracorporeal blood circuit (300), the treatment unit (200), the blood return line (302) of the extracorporeal blood circuit (300), the second circuit port (5) of the switching valve (1), the selective connection body (6) of the switching valve (1), the second transit port (3) of the switching valve (1), to arrive at the second access port (B); (ii) a second switching condition, wherein the first transit port (2) of the switching valve (1) is fluidly connected to the second circuit port (5) of the switching valve (1) to put in fluid communication the first access port (A) with the blood return line (302) of the extracorporeal blood circuit (300), the second transit port (3) of the switching valve (1) is fluidly connected to the first circuit port (4) of the switching valve (1) to put in fluid communication the second access port (B) with the withdrawal line (301) of the extracorporeal blood circuit (300), the second condition triggering a reversing mode of operation of the extracorporeal blood circuit (300) wherein blood coming from the second access port (B) flows through the second transit port (3) of the switching valve (1), the selective connection body (6) of the switching valve (1), the first circuit port (4) of the switching valve (1), the blood withdrawal line (301) of the extracorporeal blood circuit (300), the treatment unit (200), the blood return line (302) of the extracorporeal blood circuit (6), the second circuit port (5) of the switching valve (1), the selective connection body (6) of the switching valve (1), the first transit port (2) of the switching valve (1), to arrive at the first access port (A); (iii) a third switching condition, wherein the first circuit port (4) and the second circuit port (5) of the switching valve (1) are fluidly connected to each other to put in fluid communication the blood withdrawal line (301) with the blood return line (302) of the extracorporeal blood circuit (300), and the first transit port (2) and the second transit port (3) of the switching valve (1) are fluidly connected to each other to put in fluid communication the first access port (A) with the second access port (B), the third condition triggering a recirculation mode of operation of the extracorporeal blood circuit (300) wherein blood coming from the first access port (A), flows through the first transit port (2) of the switching valve (1), the selective connection body (6) of the switching valve (1), the second transit port (3) of the of the switching valve (1) to arrive to the second access port (B), and blood inside the extracorporeal blood circuit (300) is able to be re-circulated, according to a closed circuit, along the blood withdrawal line (301) of extracorporeal blood circuit (300), the treatment unit (200), the blood return line (302) of the extracorporeal blood circuit (300), the second circuit port (5) of the switching valve (1), the selective connection body (6) of the switching valve (1), the first circuit port (4) of the switching valve (1) to arrive again at the blood withdrawal line (301) of the extracorporeal blood circuit (300) or according to a direction of circulation opposite, in the third condition the selective connection body (6) of the switching valve (1) fluidly isolates the extracorporeal blood circuit (300) from the first (A) and the second access port (B) whereby these latter are not in fluid communication with the extracorporeal blood circuit (300).

In a 1^{st} ter independent aspect, which may be combined with any other previous or following aspects or portions thereof, an extracorporeal blood treatment apparatus (100) comprises: a treatment unit (200); an extracorporeal blood circuit (300) comprising a blood withdrawal line (301) connected to an inlet (201) of the treatment unit (200) and connectable to a first access port (A), opposite to the treatment unit (200), for the passage of blood inwards the extracorporeal blood circuit (300), and a blood return line (302) connected to an outlet (202) of the treatment unit (200) and connectable to a second access port (B), opposite to the treatment unit (200), for the passage of blood outwards the extracorporeal blood circuit (300); at least one switching valve (1) provided with: a first transit port (2) in fluid communication with the first access port (A); a second transit port (3) in fluid communication with the second access port (B); a first circuit port (4) in fluid communication with the blood withdrawal line (301) of the extracorporeal blood circuit (300); a second circuit port (5) in fluid communication with the blood return line (302) of the extracorporeal blood circuit (300); a selective connection body (6) provided with a first pair (7a, 7b) of through channels (7a, 7b, 8a, 8b, 9a, 9b) configured to respectively fluidly connect the first transit port (2) with the first circuit port (4) of the switching valve (1) and the second transit port (3) with the second circuit port (5) of the switching valve (1), a second pair (8a, 8b) of through channels (7a, 7b, 8a, 8b, 9a, 9b), distinct with respect to the through channels of the first pair (7a, 7b) and configured to respectively fluidly connect the first transit port (2) with the second circuit port (5) of the switching valve (1) and the second transit port (3) with the first circuit port (4) of the switching valve (1), and, a third pair (9a, 9b) of through channels (7a, 7b, 8a, 8b, 9a, 9b) distinct with respect to the through channels of the first (7a, 7b) and the second pair (8a, 8b) and configured respectively to fluidly connect the first transit port (2) of the witching valve (1) to the second transit port (3) of the switching valve (1), and the first circuit port (4) of the switching valve (1) with the second circuit port (5) of the switching valve (1), the selective connection body (6) being movable between: (i) a first switching condition, wherein the first pair (7a, 7b) of through channels (7a, 7b, 8a, 8b, 9a, 9b) fluidly connects the first transit port (2) and the second transit port (3) of the switching valve (1) respectively with the first circuit port (4) and the second circuit port (5) of the switching valve (1) to put in fluid communication the first access port (A) and the second access port (B) respectively with the blood withdrawal line (301) and the blood return line (302) of the extracorporeal blood circuit (300), the first condition triggering a normal mode of operation of the extracorporeal blood circuit (300) wherein blood coming from the first access port (A), flows through the first transit port (2) of the switching valve (1), a corresponding through channel (7a) of the first pair (7a, 7b) of the selective connection body (6) of the switching valve (1), the first circuit port (4) of the switching valve (1), the withdrawal line (301) of the extracorporeal blood circuit (300), the treatment unit (200), the blood return line (302) of the extracorporeal blood circuit (300), the second circuit port (5) of the switching valve (1), the other corresponding through channel (7b) of the first pair (7a, 7b) of the selective connection body (6) of the switching valve (1), the second transit port (3) of the switching valve (1), to arrive at the second access port (B); (ii) a second switching condition, wherein the second pair (8a, 8b) of through channels (7a, 7b, 8a, 8b, 9a, 9b) fluidly connect the first transit port (2) and the second transit port (3) of the switching valve (1) respectively with the second circuit port (5) and the first circuit port (4) of the switching valve (1), to put in fluid communication the first access port (A) with the blood return line (302) of the extracorporeal blood circuit (300) and the second access port (B) with the blood withdrawal line (301) of the extracorporeal blood circuit (300), the second condition triggering a reversing mode of operation of the extracorporeal blood circuit (300) wherein blood coming from the second access port (B) flows through the second transit port (3) of the switching valve (1), the corresponding through channel (8b) of the second pair (8a, 8b) of the selective connection body (6) of the switching valve (1), the first circuit port (4) of the switching valve (1), the blood withdrawal line (301) of the extracorporeal blood circuit (300), the treatment unit (200), the blood return line (302) of the extracorporeal blood circuit (300), the second circuit port (5) of the switching valve (1), the other corresponding through channel (8a) of the second pair (8a, 8b) of the selective connection body (6) of the switching valve (1), the first transit port (2) of the switching valve (1), to arrive at the first access port (A); (iii) a third switching condition, wherein the third pair (9a, 9b) of through channels (7a, 7b, 8a, 8b, 9a, 9b) fluidly connect the first transit port (2) with the second transit port (3) of the switching valve (1), and the first circuit port (4) with the second circuit port (5) of the switching valve (1), to respectively put in fluid communication the first access port (A) with the second access port (B) and the blood withdrawal line (301) with the blood return line (302) of the extracorporeal blood circuit (300), the third condition triggering a recirculation mode of operation of the extracorporeal blood circuit (300) wherein blood coming from the first access port (B), flows through the first transit port (2) of the switching valve (1), the corresponding through channel (9a) of the third pair (9a, 9b) of the selective connection body (6) of the switching valve (1), the second transit port (3) of the switching valve (1) to arrive to the second access port (B), and the blood inside the extracorporeal blood circuit (300) is able to be re-circulated, according to a closed circuit, along the blood withdrawal line (301) of extracorporeal blood circuit (300), the treatment unit (200), the blood return line (302) of the extracorporeal blood circuit (300), the second circuit port (5) of the switching valve (1), the other corresponding through channel (9b) of the third pair (9a, 9b) of the selective connection body (6) of the switching valve (1), the first circuit port (4) of the switching valve (1), to arrive again at the blood withdrawal line (301) of the extracorporeal blood circuit (300) or according to an opposite circulation direction, in the third condition the selective connection body (6) of the switching valve (1) fluidly isolates the extracorporeal blood circuit (300) from the first (A) and the second access port (B) whereby these latter are not in fluid communication with the extracorporeal blood circuit (300).

In a 1^{st} quater independent aspect, which may be combined with any other previous or following aspects or portions thereof, it is provided a switching valve (1) for an extracorporeal blood treatment apparatus (100) of the type comprising: a treatment unit (200); an extracorporeal blood circuit (300) comprising a blood withdrawal line (301) connected to an inlet (201) of the treatment unit (200) and connectable to a first access port (A), opposite to the treatment unit (200), for the passage of blood inwards the extracorporeal blood circuit (300), and a blood return line (301) connected to an outlet (202) of the treatment unit (200) and connectable to a second access port (B), opposite to the treatment unit (200), for the passage of blood outwards the extracorporeal blood circuit (300); the switching valve (1) being configured to be in fluid communication with the blood withdrawal line (301) and the blood return line (302) of the extracorporeal blood circuit (300) and in fluid communication with the first access port (A) and the second access port (B), the switching valve (1) being also configured to be switchable between: (i) a first switching condition, wherein the first access port (A) and the second access port (B) are respectively fluidly connected to the blood withdrawal line (301) of the extracorporeal blood circuit (300) and the blood return line (302) of the extracorporeal blood circuit (300) to trigger a normal mode of operation of the extracorporeal blood circuit (300) wherein blood coming from the first access port (A) flows through the switching valve (1), the withdrawal line (301) of the extracorporeal blood circuit (300), the treatment unit (200), the blood return line (302) of the extracorporeal blood circuit (300), the switching valve (1) again, to arrive at the second access port (B); (ii) a second switching condition, wherein the first access port (A) and the second access port (B) are respectively fluidly connected to the blood return line (302) of the extracorporeal blood circuit (300) and the blood withdrawal line (301) of the extracorporeal blood circuit (300) to trigger a reversing mode of operation of the extracorporeal blood circuit (300) wherein blood coming from the second access port (B) flows through the switching valve (1), the blood withdrawal line (301) of the extracorporeal blood circuit (300), the treatment unit (200), the blood return line (302) of the extracorporeal blood circuit (300), the switching valve (1) again, to arrive to the first access port (A); (iii) a third switching condition, wherein the first access port (A) and the second access port (B) are fluidly connected to each other and the blood withdrawal line (301) and the blood return blood line (302) of the extracorporeal blood circuit (300) are fluidly connected to each other to trigger a recirculation mode of the extracorporeal blood circuit (300) wherein the extracorporeal blood circuit (300) is not in fluid communication with the first (A) and the second access port (B).

In a 1^{st} quinquies independent aspect, which may be combined with any other previous or following aspects or portions thereof, it is provided a switching valve (1) for an extracorporeal blood treatment apparatus (100) of the type comprising: a treatment unit (200); an extracorporeal blood circuit (300) comprising a blood withdrawal line (301) connected to an inlet (201) of the treatment unit (200) and connectable to a first access port (A), opposite to the treatment unit (200), for the passage of blood inwards the extracorporeal blood circuit (300), and a blood return line (302) connected to an outlet (202) of the treatment unit (200) and connectable to a second access port (B), opposite to the treatment unit (200), for the passage of blood outwards the extracorporeal blood circuit (300); the switching valve (1) being provided with: a first transit port (2) in fluid communication with the first access port (A); a second transit port (3) in fluid communication with the second access port (B); a first circuit port (4) in fluid communication with the blood withdrawal line (301) of the extracorporeal blood circuit (300); a second circuit port (5) in fluid communication with the blood return line (302) of the extracorporeal blood circuit (300); a selective connection body (6) configured to fluidly connect distinct pairs of ports (2, 3, 4, 5) of the switching valve (1), the selective connection body (6) being movable between: (i) a first switching condition, wherein the first transit port (2) of the switching valve (1) is fluidly connected to the first circuit port (4) of the first switching valve (1) to put in fluid communication the first access port (A) with the blood withdrawal line (301) of the extracorporeal blood circuit (300), and the second transit port (3) of the switching valve (1) is fluidly connected to the second circuit port (5) of the switching valve (1) to put in fluid communication the second access port (B) with the blood return line (302) of the extracorporeal blood circuit (300), the first condition triggering a normal mode of operation of the extracorporeal blood circuit (300) wherein blood coming from the first access port (A), flows through the first transit port (2) of the switching valve (1), the selective connection body (6) of the switching valve (1), the first circuit port (4) of the switching valve (1), the withdrawal line (301) of the extracorporeal blood circuit (300), the treatment unit (200), the blood return line (302) of the extracorporeal blood circuit (300), the second circuit port (5) of the switching valve (1), the selective connection body (6) of the switching valve (1), the second transit port (3) of the switching valve (1), to arrive at the second access port (B); (ii) a second switching condition, wherein the first transit port (2) of the switching valve (1) is fluidly connected to the second circuit port (5) of the switching valve (1) to put in fluid communication the first access port (A) with the blood return line (302) of the extracorporeal blood circuit (300), the second transit port (3) of the switching valve (1) is fluidly connected to the first circuit port (4) of the switching valve (1) to put in fluid communication the second access port (B) with the withdrawal line (301) of the extracorporeal blood circuit (300), the second condition triggering a reversing mode of operation of the extracorporeal blood circuit (300) wherein blood coming from the second access port (B) flows through the second transit port (3) of the switching valve (1), the selective connection body (6) of the switching valve (1), the first circuit port (4) of the switching valve (1), the blood withdrawal line (301) of the extracorporeal blood circuit (300), the treatment unit (200), the blood return line (302) of the extracorporeal blood circuit (6), the second circuit port (5) of the switching valve (1), the selective connection body (6) of the switching valve (1), the first transit port (2) of the switching valve (1), to arrive at the first access port (A); (iii) a third switching condition, wherein the first circuit port (4) and the second circuit port (5) of the switching valve (1) are fluidly connected to each other to put in fluid communication the blood withdrawal line (301) with the blood return line (302) of the extracorporeal blood circuit (300), and the first transit port (2) and the second transit port (3) of the switching valve (1) are fluidly connected to each other to put in fluid communication the first access port (A) with the second access port (B), the third condition triggering a recirculation mode of operation of the extracorporeal blood circuit (300) wherein blood coming from the first access port (A), flows through the first transit port (2) of the switching valve (1), the selective connection body (6) of the switching valve (1), the second transit port (3) of the of the switching valve (1) to arrive to the second access port (B), and blood inside the extracorporeal blood circuit (300) is able to be circulated, according to a closed circuit, along the blood withdrawal line (301) of extracorporeal blood circuit (300), the treatment unit (200), the blood return line (302) of the extracorporeal blood circuit (300), the second circuit port (5) of the switching valve (1), the selective connection body (6) of the switching valve (1), the first circuit port (4) of the switching valve (1) to arrive again at the blood withdrawal line (301) of the extracorporeal blood circuit (300) or according to a circulation direction opposite, in the third condition the selective connection body (6) of the switching valve (1) fluidly isolates the extracorporeal blood circuit (30) from the first (A) and the second access port (B) whereby these latter are not in fluid communication with the extracorporeal blood circuit (300).

in a 1^{st} sexies independent aspect, which may be combined with any other previous or following aspects or portions thereof, it is provided a switching valve (1) for an extracorporeal blood treatment apparatus (100) of the type comprising: a treatment unit (200); an extracorporeal blood circuit (300) comprising a blood withdrawal line (301) connected to an inlet (201) of the treatment unit (200) and connectable to a first access port (A), opposite to the treatment unit (200), for the passage of blood inwards the extracorporeal blood circuit (300), and a blood return line (302) connected to an outlet (202) of the treatment unit (200) and connectable to a second access port (B), opposite to the treatment unit (200), for the passage of blood outwards the extracorporeal blood circuit (300); the switching valve (1) being provided with: a first transit port (2) in fluid communication with the first access port (A); a second transit port (3) in fluid communication with the second access port (B); a first circuit port (4) in fluid communication with the blood withdrawal line (301) of the extracorporeal blood circuit (300); a second circuit port (5) in fluid communication with the blood return line (302) of the extracorporeal blood circuit (300); a selective connection body (6) provided with a first pair (7a, 7b) of through channels (7a, 7b, 8a, 8b, 9a, 9b) configured to respectively fluidly connect the first transit port (2) with the first circuit port (4) of the switching valve (1) and the second transit port (3) with the second circuit port (5) of the switching valve (1), a second pair (8a, 8b) of through channels (7a, 7b, 8a, 8b, 9a, 9b), distinct with respect to the through channels of the first pair (7a, 7b) and configured to respectively fluidly connect the first transit port (2) with the second circuit port (5) of the switching valve (1) and the second transit port (3) with the first circuit port (4) of the switching valve (1), and, a third pair (9a, 9b) of through channels (7a, 7b, 8a, 8b, 9a, 9b) distinct with respect to the through channels of the first (7a, 7b) and the second pair (8a, 8b) and configured respectively to fluidly connect the first transit port (2) of the witching valve (1) to the second transit port (3) of the switching valve (1), and the first circuit port (4) of the switching valve (1) with the second circuit port (5) of the switching valve (1), the selective connection body (6) being movable between: (i) a first switching condition, wherein the first pair (7a, 7b) of through channels (7a, 7b, 8a, 8b, 9a, 9b) fluidly connects the first transit port (2) and the second transit port (3) of the switching valve (1) respectively with the first circuit port (4) and the second circuit port (5) of the switching valve (1) to put in fluid communication the first access port (A) and the second access port (B) respectively with the blood withdrawal line (301) and the blood return line (302) of the extracorporeal blood circuit (300), the first condition triggering a normal mode of operation of the extracorporeal blood circuit (300) wherein blood coming from the first access port (A), flows through the first transit port (2) of the switching valve (1), a corresponding through channel (7a) of the first pair (7a, 7b) of the selective connection body (6) of the switching valve (1), the first circuit port (4) of the switching valve (1), the withdrawal line (301) of the extracorporeal blood circuit (300), the treatment unit (200), the blood return line (302) of the extracorporeal blood circuit (300), the second circuit port (5) of the switching valve (1), the other corresponding through channel (7b) of the first pair (7a, 7b) of the selective connection body (6) of the switching valve (1), the second transit port (3) of the switching valve (1), to arrive at the second access port (B); (ii) a second switching condition, wherein the second pair (8a, 8b) of through channels (7a, 7b, 8a, 8b, 9a, 9b) fluidly connect the first transit port (2) and the second transit port (3) of the switching valve (1) respectively with the second circuit port (5) and the first circuit port (4) of the switching valve (1) to put in fluid communication the first access port (A) with the blood return line (302) of the extracorporeal blood circuit (300) and the second access port (302) with the blood withdrawal line (301) of the extracorporeal blood circuit (300), the second condition triggering a reversing mode of operation of the extracorporeal blood circuit (300) wherein blood coming from the second access port (B) flows through the second transit port (3) of the switching valve (1), the corresponding through channel (8b) of the second pair (8a, 8b) of the selective connection body (6) of the switching valve (1), the first circuit port (4) of the switching valve (1), the blood withdrawal line (301) of the extracorporeal blood circuit (300), the treatment unit (200), the blood return line (302) of the extracorporeal blood circuit (300), the second circuit port (5) of the switching valve (1), the other corresponding through channel (8a) of the second pair (8a, 8b) of the selective connection body (6) of the switching valve (1), the first transit port (2) of the switching valve (1), to arrive at the first access port (A); (iii) a third switching condition, wherein the third pair (9a, 9b) of through channels (7a, 7b, 8a, 8b, 9a, 9b) fluidly connect the first transit port (2) with the second transit port (3) of the switching valve (1), and the first circuit port (4) with the second circuit port (5) of the switching valve (1), to respectively put in fluid communication the first access port (A) with the second access port (B) and the blood withdrawal line (301) with the blood return line (302) of the extracorporeal blood circuit (300), the third condition triggering a recirculation mode of operation of the extracorporeal blood circuit (300) wherein blood coming from the first access port (B), flows through the first transit port (2) of the switching valve (1), the corresponding through channel (9a) of the third pair (9a, 9b) of the selective connection body (6) of the switching valve (1), the second transit port (3) of the switching valve (1) to arrive to the second access port (B), and the blood inside the extracorporeal blood circuit (300) is able to be circulated, according to a closed circuit, along the blood withdrawal line (301) of extracorporeal blood circuit (300), the treatment unit (200), the blood return line (302) of the extracorporeal blood circuit (300), the second circuit port (5) of the switching valve (1), the other corresponding through channel (9b) of the third pair (9a, 9b) of the selective connection body (6) of the switching valve (1), the first circuit port (4) of the switching valve (1), to arrive again at the blood withdrawal line (301) of the extracorporeal blood circuit (300) or according to an opposite circulation direction, in the third condition the selective connection body (6) of the switching valve (1) fluidly isolates the extracorporeal blood circuit (300) from the first (A) and the second access port (B) whereby these latter are not in fluid communication with the extracorporeal blood circuit (300).

In a 1^{st} septies independent aspect, which may be combined with any other previous or following aspects or portions thereof, it is provided the use of a switching valve (1) in, or with, an extracorporeal blood treatment apparatus (100) of the type comprising: a treatment unit (200); an extracorporeal blood circuit (300) comprising a blood withdrawal line (301) connected to an inlet (201) of the treatment unit (200) and connectable to a first access port (A), opposite to the treatment unit (200), for the passage of blood inwards the extracorporeal blood circuit (300), and a blood return line (302) connected to an outlet (202) of the treatment unit (200) and connectable to a second access port (B), opposite to the treatment unit (200), for the passage of blood outwards the extracorporeal blood circuit (300); the switching valve (1) being configured to be in fluid communication with the blood withdrawal line (301) and the blood return line (302) of the extracorporeal blood circuit (300) and in fluid communication with the first access port (A) and the second access port (B), the switching valve (1) being also configured to be switchable between: (i) a first switching condition, wherein the first access port (A) and the second access port (B) are respectively fluidly connected to the blood withdrawal line (301) of the extracorporeal blood circuit (300) and the blood return line (301) of the extracorporeal blood circuit (300) to trigger a normal mode of operation of the extracorporeal blood circuit (300) wherein blood coming from the first access port (A) flows through the switching valve (1), the withdrawal line (301) of the extracorporeal blood circuit (300), the treatment unit (200), the blood return line (302) of the extracorporeal blood circuit (300), the switching valve (1) again, to arrive at the second access port (B); (ii) a second switching condition, wherein the first access port (A) and the second access port (B) are respectively fluidly connected to the blood return line (302) of the extracorporeal blood circuit (300) and the blood withdrawal line (301) of the extracorporeal blood circuit (300) to trigger a reversing mode of operation of the extracorporeal blood circuit (300) wherein blood coming from the second access port (B) flows through the switching valve (1), the blood withdrawal line (301) of the extracorporeal blood circuit (300), the treatment unit (200), the blood return line (302) of the extracorporeal blood circuit (300), the switching valve (1) again, to arrive to the first access port (A); (iii) a third switching condition, wherein the first access port (B) and the second access port (B) are fluidly connected to each other and the blood withdrawal line (301) and the blood return blood line (302) of the extracorporeal blood circuit (300) are fluidly connected to each other to trigger a recirculation mode of the extracorporeal blood circuit (300) wherein the extracorporeal blood circuit (300) is not in fluid communication with the first (A) and the second access port (B).

In a 1^{st} octies independent aspect, which may be combined with any other previous or following aspects or portions thereof, it is provided the use of a switching valve (1) in or with an extracorporeal blood treatment apparatus (100) of the type comprising: a treatment unit (200); an extracorporeal blood circuit (300) comprising a blood withdrawal line (301) connected to an inlet (201) of the treatment unit (200) and connectable to a first access port (A), opposite to the treatment unit (200), for the passage of blood inwards the extracorporeal blood circuit (300), and a blood return line (302) connected to an outlet (202) of the treatment unit (200) and connectable to a second access port (B), opposite to treatment unit (200), for the passage of blood outwards the extracorporeal blood circuit (300); the switching valve (1) being provided with: a first transit port (2) in fluid communication with the first access port (A); a second transit port (3) in fluid communication with the second access port (B); a first circuit port (4) in fluid communication with the blood withdrawal line (301) of the extracorporeal blood circuit (300); a second circuit port (5) in fluid communication with the blood return line (302) of the extracorporeal blood circuit (300); a selective connection body (6) configured to fluidly connect distinct pairs of ports (2, 3, 4, 5) of the switching valve (1), the selective connection body (6) being movable between: (i) a first switching condition, wherein the first transit port (2) of the switching valve (1) is fluidly connected to the first circuit port (4) of the first switching valve (1) to put in fluid communication the first access port (A) with the blood withdrawal line (301) of the extracorporeal blood circuit (300), and the second transit port (3) of the switching valve (1) is fluidly connected to the second circuit port (5) of the switching valve (1) to put in fluid communication the second access port (B) with the blood return line (302) of the extracorporeal blood circuit (300), the first condition triggering a normal mode of operation of the extracorporeal blood circuit (300) wherein blood coming from the first access port (A), flows through the first transit port (2) of the switching valve (1), the selective connection body (6) of the switching valve (1), the first circuit port (4) of the switching valve (1), the withdrawal line (301) of the extracorporeal blood circuit (300), the treatment unit (200), the blood return line (302) of the extracorporeal blood circuit (300), the second circuit port (5) of the switching valve (1), the selective connection body (6) of the switching valve (1), the second transit port (3) of the switching valve (1), to arrive at the second access port (B); (ii) a second switching condition, wherein the first transit port (2) of the switching valve (1) is fluidly connected to the second circuit port (5) of the switching valve (1) to put in fluid communication the first access port (A) with the blood return line (302) of the extracorporeal blood circuit (300), the second transit port (3) of the switching valve (1) is fluidly connected to the first circuit port (4) of the switching valve (1) to put in fluid communication the second access port (B) with the withdrawal line (301) of the extracorporeal blood circuit (300), the second condition triggering a reversing mode of operation of the extracorporeal blood circuit (300) wherein blood coming from the second access port (B) flows through the second transit port (3) of the switching valve (1), the selective connection body (6) of the switching valve (1), the first circuit port (4) of the switching valve (1), the blood withdrawal line (301) of the extracorporeal blood circuit (300), the treatment unit (200), the blood return line (302) of the extracorporeal blood circuit (6), the second circuit port (5) of the switching valve (1), the selective connection body (6) of the switching valve (1), the first transit port (2) of the switching valve (1), to arrive at the first access port (A); (iii) a third switching condition, wherein the first circuit port (4) and the second circuit port (5) of the switching valve (1) are fluidly connected to each other to put in fluid communication the blood withdrawal line (301) with the blood return line (302) of the extracorporeal blood circuit (300), and the first transit port (2) and the second transit port (3) of the switching valve (1) are fluidly connected to each other to put in fluid communication the first access port (A) with the second access port (B), the third condition triggering a recirculation mode of operation of the extracorporeal blood circuit (300) wherein blood coming from the first access port (A), flows through the first transit port (2) of the switching valve (1), the selective connection body (6) of the switching valve (1), the second transit port (3) of the of the switching valve (1) to arrive to the second access port (B), and blood inside the extracorporeal blood circuit (300) is able to be circulated, according to a closed circuit, along the blood withdrawal line (301) of extracorporeal blood circuit (300), the treatment unit (200), the blood return line (302) of the extracorporeal blood circuit (300), the second circuit port (5) of the switching valve (1), the selective connection body (6) of the switching valve (1), the first circuit port (4) of the switching valve (1) to arrive again at the blood withdrawal line (301) of the extracorporeal blood circuit (300) or according to a circulation direction opposite, in the third condition the selective connection body (6) of the switching valve (1) fluidly isolates the extracorporeal blood circuit (30) from the first (A) and the second access port (B) whereby these latter are not in fluid communication with the extracorporeal blood circuit (300).

In a 1^{st} novies independent aspect, which may be combined with any other previous or following aspects or portions thereof, it is provided the use of a switching valve (1) in or with an extracorporeal blood treatment apparatus (100) of the type comprising: a treatment unit (200); an extracorporeal blood circuit (300) comprising a blood withdrawal line (301) connected to an inlet (201) of the treatment unit (200) and connectable to a first access port (A), opposite to the treatment unit (200), for the passage of blood inwards the extracorporeal blood circuit (300), and a blood return line (302) connected to an outlet (202) of the treatment unit (200) and connectable to a second access port (B), opposite to the treatment unit (200), for the passage of blood outwards the extracorporeal blood circuit (300); the switching valve (1) being provided with: a first transit port (2) in fluid communication with the first access port (A); a second transit port (3) in fluid communication with the second access port (B); a first circuit port (4) in fluid communication with the blood withdrawal line (301) of the extracorporeal blood circuit (300); a second circuit port (5) in fluid communication with the blood return line (302) of the extracorporeal blood circuit (300); a selective connection body (6) provided with a first pair (7a, 7b) of through channels (7a, 7b, 8a, 8b, 9a, 9b) configured to respectively fluidly connect the first transit port (2) with the first circuit port (4) of the switching valve (1) and the second transit port (3) with the second circuit port (5) of the switching valve (1), a second pair (8a, 8b) of through channels (7a, 7b, 8a, 8b, 9a, 9b), distinct with respect to the through channels of the first pair (7a, 7b) and configured to respectively fluidly connect the first transit port (2) with the second circuit port (5) of the switching valve (1) and the second transit port (3) with the first circuit port (4) of the switching valve (1), and, a third pair (9a, 9b) of through channels (7a, 7b, 8a, 8b, 9a, 9b) distinct with respect to the through channels of the first (7a, 7b) and the second pair (8a, 8b) and configured respectively to fluidly connect the first transit port (2) of the witching valve (1) to the second transit port (3) of the switching valve (1), and the first circuit port (4) of the switching valve (1) with the second circuit port (5) of the switching valve (1), the selective connection body (6) being movable between: (i) a first switching condition, wherein the first pair (7a, 7b) of through channels (7a, 7b, 8a, 8b, 9a, 9b) fluidly connects the first transit port (2) and the second transit port (3) of the switching valve (1) respectively with the first circuit port (4) and the second circuit port (5) of the switching valve (1) to put in fluid communication the first access port (A) and the second access port (B) respectively with the blood withdrawal line (301) and the blood return line (302) of the extracorporeal blood circuit (300), the first condition triggering a normal mode of operation of the extracorporeal blood circuit (300) wherein blood coming from the first access port (A), flows through the first transit port (2) of the switching valve (1), a corresponding through channel (7a) of the first pair (7a, 7b) of the selective connection body (6) of the switching valve (1), the first circuit port (4) of the switching valve (1), the withdrawal line (301) of the extracorporeal blood circuit (300), the treatment unit (200), the blood return line (302) of the extracorporeal blood circuit (300), the second circuit port (5) of the switching valve (1), the other corresponding through channel (7b) of the first pair (7a, 7b) of the selective connection body (6) of the switching valve (1), the second transit port (3) of the switching valve (1), to arrive at the second access port (B); (ii) a second switching condition, wherein the second pair (8a, 8b) of through channels (7a, 7b, 8a, 8b, 9a, 9b) fluidly connect the first transit port (2) and the second transit port (3) of the switching valve (1) respectively with the second circuit port (5) and the first circuit port (4) of the switching valve (1) to put in fluid communication the first access port (A) with the blood return line (302) of the extracorporeal blood circuit (300) and the second access port (302) with the blood withdrawal line (301) of the extracorporeal blood circuit (300), the second condition triggering a reversing mode of operation of the extracorporeal blood circuit (300) wherein blood coming from the second access port (B) flows through the second transit port (3) of the switching valve (1), the corresponding through channel (8b) of the second pair (8a, 8b) of the selective connection body (6) of the switching valve (1), the first circuit port (4) of the switching valve (1), the blood withdrawal line (301) of the extracorporeal blood circuit (300), the treatment unit (200), the blood return line (302) of the extracorporeal blood circuit (300), the second circuit port (5) of the switching valve (1), the other corresponding through channel (8a) of the second pair (8a, 8b) of the selective connection body (6) of the switching valve (1), the first transit port (2) of the switching valve (1), to arrive at the first access port (A); (iii) a third switching condition, wherein the third pair (9a, 9b) of through channels (7a, 7b, 8a, 8b, 9a, 9b) fluidly connect the first transit port (2) with the second transit port (3) of the switching valve (1), and the first circuit port (4) with the second circuit port (5) of the switching valve (1), to respectively put in fluid communication the first access port (A) with the second access port (B) and the blood withdrawal line (301) with the blood return line (302) of the extracorporeal blood circuit (300), the third condition triggering a recirculation mode of operation of the extracorporeal blood circuit (300) wherein blood coming from the first access port (B), flows through the first transit port (2) of the switching valve (1), the corresponding through channel (9a) of the third pair (9a, 9b) of the selective connection body (6) of the switching valve (1), the second transit port (3) of the switching valve (1) to arrive to the second access port (B), and the blood inside the extracorporeal blood circuit (300) is able to be circulated, according to a closed circuit, along the blood withdrawal line (301) of extracorporeal blood circuit (300), the treatment unit (200), the blood return line (302) of the extracorporeal blood circuit (300), the second circuit port (5) of the switching valve (1), the other corresponding through channel (9b) of the third pair (9a, 9b) of the selective connection body (6) of the switching valve (1), the first circuit port (4) of the switching valve (1), to arrive again at the blood withdrawal line (301) of the extracorporeal blood circuit (300) or according to an opposite circulation direction, in the third condition the selective connection body (6) of the switching valve (1) fluidly isolates the extracorporeal blood circuit (300) from the first (A) and the second access port (B) whereby these latter are not in fluid communication with the extracorporeal blood circuit (300).

In a 2^{nd} aspect according to any one of the previous aspects, the switching valve (1) is provided with: a first transit port (2) in fluid communication with the first access port (A); a second transit port (3) in fluid communication with the second access port (B); a first circuit port (4) in fluid communication with the blood withdrawal line (301) of the extracorporeal blood circuit (300); a second circuit port (6) in fluid communication with the blood return line (302) of the extracorporeal blood circuit (300); a selective connection body (6) configured to fluidly connect distinct pairs of ports (2, 3, 4, 5) of the switching valve (1).

In a 3^{rd} aspect according to the previous aspect, the selective connection body (6) is movable between: (i) a first switching condition, wherein the first transit port (2) of the switching valve (1) is fluidly connected to the first circuit port (4) of the first switching valve (1) to put in fluid communication the first access port (A) with the blood withdrawal line (301) of the extracorporeal blood circuit (300), and the second transit port (3) of the switching valve (1) is fluidly connected to the second circuit port (5) of the switching valve (1) to put in fluid communication the second access port (B) with the blood return line (302) of the extracorporeal blood circuit (300), the first condition triggering a normal mode of operation of the extracorporeal blood circuit (300) wherein blood coming from the first access port (A), flows through the first transit port (2) of the switching valve (1), the selective connection body (6) of the switching valve (1), the first circuit port (4) of the switching valve (1), the withdrawal line (301) of the extracorporeal blood circuit (300), the treatment unit (200), the blood return line (302) of the extracorporeal blood circuit (300), the second circuit port (5) of the switching valve (1), the selective connection body (6) of the switching valve (1), the second transit port (3) of the switching valve (1), to arrive at the second access port (B); (ii) a second switching condition, wherein the first transit port (2) of the switching valve (1) is fluidly connected to the second circuit port (5) of the switching valve (1) to put in fluid communication the first access port (A) with the blood return line (301) of the extracorporeal blood circuit (300), the second transit port (3) of the switching valve (1) is fluidly connected to the first circuit port (4) of the switching valve (1) to put in fluid communication the second access port (B) with the withdrawal line (301) of the extracorporeal blood circuit (300), the second condition triggering a reversing mode of operation of the extracorporeal blood circuit (300) wherein blood coming from the second access port (B) flows through the first transit port (2) of the switching valve (1), the selective connection body (6) of the switching valve (1), the first circuit port (4) of the switching valve (1), the blood withdrawal line (301) of the extracorporeal blood circuit (300), the treatment unit (200), the blood return line (302) of the extracorporeal blood circuit (300), the second circuit port (5) of the switching valve (1), the selective connection body (6) of the switching valve (1), the first transit port (2) of the switching valve (1), to arrive at the first access port (A); (iii) a third switching condition, wherein the first circuit port (4) and the second circuit port (5) of the switching valve (1) are fluidly connected to each other to put in fluid communication the blood withdrawal line (301) with the blood return line (302) of the extracorporeal blood circuit (300), and the first transit port (2) and the second transit port (3) of the switching valve (1) are fluidly connected to each other to put in fluid communication the first access port (A) with the second access port (B), the third condition triggering a recirculation mode of operation of the extracorporeal blood circuit (6) wherein blood coming from the first access port (A), flows through the first transit port (2) of the switching valve (1), the selective connection body (6) of the switching valve (1), the second transit port (3) of the of the switching valve (1) to arrive to the second access port (B), and blood inside the extracorporeal blood circuit (300) is able to be circulated, according to a closed circuit, along the blood withdrawal line (301) of extracorporeal blood circuit (300), the treatment unit (200), the blood return line (302) of the extracorporeal blood circuit (300), the second circuit port (5) of the switching valve (1), the selective connection body (6) of the switching valve (1), first circuit port (4) of the switching valve (1) to arrive again at the blood withdrawal line (301) of the extracorporeal blood circuit (300) or according to a circulation direction opposite, in the third condition the selective connection body (6) of the switching valve fluidly isolates the extracorporeal blood circuit (300) from the first (A) and the second access port (B) whereby theses latter are not in fluid communication with the extracorporeal blood circuit (6).

In a 4^{th} aspect according to any one of the previous aspects, the selective connection body (6) comprises a first pair (7a, 7b) of through channels (7a, 7b, 8a, 8b, 9a, 9b) configured to respectively fluidly connect the first transit port (2) with the first circuit port (4) of the switching valve (1) and the second transit port (3) with the second circuit port (5) of the switching valve (1), a second pair (8a, 8b) of through channels (7a, 7b, 8a, 8b, 9a, 9b), distinct with respect to the through channels (7a, 7b, 8a, 8b, 9a, 9b) of the first pair (7a, 7b) and configured to respectively fluidly connect the first transit port (2) with the second circuit port (5) of the switching valve (1) and the second transit port (3) with the first circuit port (4) of the switching valve (1), and, a third pair (9a, 9b) of through channels (7a, 7b, 8a, 8b, 9a, 9b) distinct with respect to the through channels (7a, 7b, 8a, 8b, 9a, 9b) of the first (7a, 7b) and the second pairs (8a, 8b) and configured respectively to fluidly connect the first transit port (2) of the witching valve (1) to the second transit port (3) of the switching valve (1), and the first circuit port (4) of the switching valve (1) with the second circuit port (5) of the switching valve (1).

In a 5^{th} aspect according to the previous aspect the selective connection body (6) is movable between: (i) a first switching condition, wherein the first pair (7a, 7b) of through channels (7a, 7b, 8a, 8b, 9a, 9b) fluidly connects the first transit port (2) and the second transit port (3) of the switching valve (1) respectively with the first circuit port (4) and the second circuit port (5) of the switching valve (1) to put in fluid communication the first access port (A) and the second access port (B) respectively with the blood withdrawal line (301) and the return line (302) of the extracorporeal blood circuit (300), the first condition triggering a normal mode of operation of the extracorporeal blood circuit (300) wherein blood coming from the first access port (A), flows through the first transit port (2) of the switching valve (1), a corresponding through channel (7a) of the first pair (7a, 7b) of the selective connection body (6) of the switching valve (1), the first circuit port (4) of the switching valve (1), the withdrawal line (301) of the extracorporeal blood circuit (300), the treatment unit (200), the blood return line (302) of the extracorporeal blood circuit (300), the second circuit port (5) of the switching valve (1), the other corresponding through channel (7b) of the first pair (7a, 7b) of the selective connection body (6) of the switching valve (1), the second transit port (3) of the switching valve (1), to arrive at the second access port (B); (ii) a second switching condition, wherein the second pair (8a, 8b) of through channels (7a, 7b, 8a, 8b, 9a, 9b) fluidly connect the first transit port (2) and the second transit port (3) of the switching valve (1) respectively with the second circuit port (5) and the first circuit port (4) of the switching valve (1) to put in fluid communication the first access port (A) with the blood return line (301) of the extracorporeal blood circuit (300) and the second access port (B) with the blood withdrawal line (301) of the extracorporeal blood circuit (300), the second condition triggering a reversing mode of operation of the extracorporeal blood circuit (300) wherein blood coming from the second access port (B) flows through the second transit port (3) of the switching valve (1), the corresponding through channel (8b) of the second pair (8a, 8b) of the selective connection body (6) of the switching valve (1), the first circuit port (4) of the switching valve (1), the blood withdrawal line (301) of the extracorporeal blood circuit (300), the treatment unit (200), the blood return line (302) of the extracorporeal blood circuit (300), the second circuit port (5) of the switching valve (1), the other corresponding through channel (8a) of the second pair (8a, 8b) of the selective connection body (6) of the switching valve (1), the first transit port (2) of the switching valve (1), to arrive at the first access port (A); (iii) a third switching condition, wherein the third pair (9a, 9b) of through channels (7a, 7b, 8a, 8b, 9a, 9b) fluidly connect the first transit port (2) with the second transit port (3) of the switching valve (1), and the first circuit port (4) with the second circuit port (5) of the switching valve (1), to respectively put in fluid communication the first access port (A) with the second access port (B) and the blood withdrawal line (301) with the blood return line (302) of the extracorporeal blood circuit (300), the third condition triggering a recirculation mode of operation of the extracorporeal blood circuit (300) wherein blood coming from the first access port (A), flows through the first transit port (2) of the switching valve (1), the corresponding through channel (9a) of the third pair (9a, 9b) of the selective connection body (6) of the switching valve (1), the second transit port (3) of the switching valve (1) to arrive to the second access port (B), and blood inside the extracorporeal blood circuit (300) is able to be circulated, according to a closed circuit, along the blood withdrawal line (301) of extracorporeal blood circuit (300), the treatment unit (200), the blood return line (302) of the extracorporeal blood circuit (300), the second circuit port (5) of the switching valve (1), the other corresponding through channel (9b) of the third pair (9a, 9b) of the selective connection body (6) of the switching valve (1), the first circuit port (4) of the switching valve (1), to arrive again at the blood withdrawal line (301) of the extracorporeal blood circuit (300) or according to an opposite circulation direction, in the third condition the selective connection body (6) of the switching valve (1) fluidly isolates the extracorporeal blood circuit (300) from the first (A) and the second access port (B) whereby these latter are not in fluid communication with the extracorporeal blood circuit (300).

In a 6^{th} aspect according to any one of the previous aspects, the switching valve (1) comprises a housing (10) defining an inner chamber (10a), the first transit port (2), the second transit port (3), the first circuit port (4) and the second circuit port (5) being defined on and/or through the housing (10) for being in communication with the inner chamber (10a) of the latter.

In a 7^{th} aspect according to the previous aspect, each port (2, 3, 4, 5) of the switching valve (1) is provided with a tubular fitting (2a, 3a, 4a, 5a) protruding externally from the housing (10) of the switching valve (1).

In an 8^{th} aspect according to any one of the two previous aspects, the first and the second transit ports (2, 3) of the switching valve (1) are positioned at different levels or lying planes with respect to the levels or lying plains of the first and the second circuit ports (4, 5) of the switching valve (1).

In a 9^{th} aspect according to any one of the three previous aspects, each port (2, 3, 4, 5) of the switching valve (1) is positioned at a corresponding level or lying plane different with respect to the levels or lying planes of any one of the other ports (2, 3, 4, 5).

In a 10^{th} aspect according to any one of the four previous aspects, the first and the second transit ports (2, 3) of the switching valve (1) diverge from each other away from the housing (10) of the switching valve (1).

In an 11^{th} aspect according to the 10^{th} aspect when depending on the 7^{th} aspect or any one of the previous aspects from the 8^{th} aspect to the 9^{th} aspect when depending on the 7^{th} aspect, the tubular fittings (2a, 3a) corresponding to the first (2) and the second transit port (3) of the switching valve (1) diverge from each other, away from the housing (10) of the switching valve (1).

In a 12^{th} aspect according to any one of the six previous aspects, the first and the second circuit ports (4, 5) of the switching valve (1) diverge from each other away from the housing (10) of the switching valve (1).

In a 13^{th} aspect according to the 12^{th} aspect when depending on the 7^{th} aspect or any one of the previous aspects from the 8^{th} aspect to the 11^{th} aspect when depending on the 7^{th} aspect, the tubular fittings (4a, 5a) corresponding to the first (4) and the second circuit port (5) of the switching valve (1) diverge from each other, away from the housing (10) of the switching valve (1).

In a 14^{th} aspect according to any one of the previous aspects, the switching valve (1) is substantially cylindrical.

in a 15^{th} aspect according to any one of the previous aspects from the 6^{th} aspect to the 13^{th} aspect or the previous aspect when depending on any one of the aspects from the 6^{th} aspect to the 13^{th} aspect, the housing (10) of the switching valve (1) is substantially cylindrical.

In a 16^{th} aspect according to any one of the previous aspects, the housing (10) of the switching valve (1) comprises: a first wall (11), in particular substantially circular; a second wall (12), in particular substantially cylindrical, transversally developing from the first wall (11); and, a third wall (13), in particular substantially circular, transversally engaged to the second wall (12), on the opposite side to the first wall (11).

In a 17^{th} aspect according to the previous aspect, the first (2) and second transit port (3) of the switching valve (1) are positioned close to the third wall (13) of the housing (10) of the switching valve (1).

In a 18^{th} aspect according to any one of the two previous aspects, the first (4) and the second circuit port (5) of the switching valve (1) are positioned close to the first wall (11) of the housing (10) of the switching valve (1).

In a 19^{th} aspect according to the 14^{th} aspect or any one of the aspects from the 15^{th} aspect to the 18^{th} aspect when depending on the 14^{th} aspect, at least one of the first and second transit port (2, 3) of the switching valve (1) has a corresponding longitudinal development axis lying on a radial plane of the switching valve (1) on which a longitudinal development axis of at least one of the first and the second circuit port (4, 5) lies.

In a 20^{th} aspect according to the 14^{th} aspect or any one of the aspects from the 15^{th} aspect to the 19^{th} aspect when depending on the 14^{th} aspect, each of the transit ports (2, 3) of the switching valve (1) has a corresponding longitudinal axis lying on a radial plane of the switching valve (1) on which a longitudinal development axis of a corresponding circuit port (4, 5) of the switching valve (1) lies.

In a 21^{st} aspect according to any one of the seven previous aspects, the transit ports (2, 3) of the switching valve (1) are positioned at an angle to each other comprised between 10° and 100°, in particular comprised between 20° and 90°, more in particular comprised between 30° ad 80°, optionally comprised between 50° and 70°, more optionally around 60°.

In a 22^{nd} aspect according to any one of the eight previous aspects, the circuit ports (4, 5) of the switching valve (1) are positioned at an angle to each other comprised between 10° and 100°, in particular comprised between 20° and 90°, more in particular comprised between 30° ad 80°, optionally comprised between 50° and 70°, more optionally around 60°.

In a 23^{rd} aspect according to the 14^{th} aspect or any one of the previous aspects from the 15^{th} aspect to the 22^{nd} aspect when depending on the 14^{th} aspect, the inner chamber (10a) is substantially cylindrical.

In 24^{th} aspect according to the previous aspect when depending on the 16^{th} aspect or any one of the aspects 17^{th} to 22^{nd} aspect when depending on the 16^{th} aspect, the inner chamber (10a) of the switching valve (1) is defined between the first wall (11), the second wall (12) and the third wall (13) of the housing (10).

In a 25^{th} aspect according to the 6^{th} aspect or any one of the previous aspects from the 7^{th} aspect to the 24^{th} aspect when depending on the 6^{th} aspect, the selective connection body (6) of the switching valve (1) operatively occupies the inner chamber (10a) of the housing (10) of the switching valve (1).

In a 26^{th} aspect according to any one of the previous aspects, the selective connection body (6) of the switching valve (1) is cylindrical.

In a 27^{th} aspect according to any one of the previous aspects, the selective connection body (6) of the switching valve (1) comprises a first wall (6a), in particular substantially circular, a second wall (6b), in particular substantially cylindrical, transversally developing from the first wall (6a), and a third wall (6c), in particular substantially circular transversally developing from the second wall (6b) and facing away from the first wall (6a).

In a 28^{th} aspect according to any one of the three previous aspects when depending on the 14^{th} aspect or the 15^{th} aspect or any one of the previous aspects when depending on the 14^{th} aspect to the 15^{th} aspect, the selective connection body (6) of the switching valve (1) rotationally occupies the inner chamber (10a) of the housing (10) of the switching valve (1).

In a 29^{th} aspect according to the 27^{th} aspect or the 28^{th} aspect when depending on 16^{th} aspect or any one of the previous aspects depending on the 16^{th} aspect, the first wall (6a), the second wall (6b) and the third wall (6c) of the selective connection body (6) of the switching valve (1) respectively face internally at the first wall (11), the second wall (12) and the third wall (13) of the housing (10) of the switching valve (1).

In a 30^{th} aspect according to any one of the previous aspects, the selective connection body (6) of the switching valve (1) is provided with at least one transmission shaft (6d) engageable, directly or indirectly, to an actuator device able to move the transmission shaft (6d) and the selective connection body (6) of the switching valve (1).

In a 31^{st} aspect according to the previous aspect, the transmission shaft (6d) protrudes from the selective connection body (6) of the switching valve (1).

In a 32^{nd} aspect according to the two previous aspects when the aspect 30^{th} depends on the 27^{th} aspect or any one of the previous aspects depending on the aspect 27^{th}, the transmission shaft (6d) develops from the third wall (6c) of the selective connection body (6) of the switching valve (1).

In a 33^{rd} aspect according to the previous aspect, the transmission shaft (6d) develops along a longitudinal axis (X) of the selective connection body (6) of the switching valve (1).

In a 34^{th} aspect according to any one of the five previous aspects, the transmission shaft (6d) develops through a corresponding through opening (10b) cut out in the housing (10).

In a 35^{th} aspect according to the previous aspect when depending on the 32^{nd} aspect or the 33^{rd} aspect, the through opening (10b) of the housing (10) of the switching valve (1) crossed by the transmission shaft (6d) of the corresponding selective connection body (6) is localized at the third wall (13) of the housing (10), in particular in the center of the third wall (13) of the housing (10), at least one seal (10c), in particular two or more seals, optionally O-ring, is interposed between the third wall (13) of the housing (10) of the switching valve (1) and the transmission shaft (6d) of the switching valve (1) in order isolate the inner chamber (10a) of the housing (10) from the outside in correspondence of the through opening (10b).

In a 36^{th} aspect according to any one of the previous aspects each through channel (7a, 7b, 8a, 8b, 9a, 9b) of the selective connection body (6) of the switching valve (1) has a circular cross-section profile.

In a 37^{th} aspect according to the 27^{th} aspect or any one of the previous aspects depending on the aspect 27^{th}, each through channel (7a, 7b, 8a, 8b, 9a, 9b) of the selective connection body (6) of the switching valve (1) comprises two fluidic connection ends (7a', 7a", 7b', 7b", 8a', 8a", 8b', 8b", 9a', 9a", 9b', 9b") localized in correspondence of the second wall (6b) to provide a fluid communication with a corresponding pair of ports (2, 3, 4, 5) of the housing (10), one fluidic connection end (7a', 7b', 8a', 8b', 9a', 9b') defining an inlet for a fluid, while the other fluidic connection end (7a", 7b", 8a", 8b", 9a", 9b") defining an outlet for the same fluid.

In a 38^{th} aspect according to any one of the previous aspects, the through channels of the first pair (7a, 7b) of the selective connection body (6) develop alongside each other, without intersecting.

In a 39^{th} aspect according to any one of the previous aspects, a median plane (M), in particular a radial median plane (M), of the selective connection body (6) of the switching valve (1), is interposed between the the through channels of the first pair (7a, 7b) of the selective connection body (6).

In a 40^{th} aspect according to any one of the previous aspects, the through channels of the first pair (7a, 7b) of the selective connection body (6) have a substantially rectilinear development.

In a 41^{st} aspect according to the 27^{th} aspect or any one of the previous aspects from the 28^{th} aspect to the 40^{th} aspect when depending on the 27^{th} aspect, the through channels of the first pair (7a, 7b) of the selective connection body (6) have a development inclined with respect to the walls (6a, 6b 6c) of the selective connection body (6).

In a 42^{nd} aspect according to any one of the previous aspects, the through channels of the second pair (8a, 8b) of the selective connection body (6) cross a same median plane (M), optionally a same radial median plane (M), of the latter.

In a 43^{rd} aspect according to any one of the previous aspects, the through channels of the second pair (8a, 8b) of the selective connection body (6) develop at different levels of the latter, according to respective crossing directions.

In a 44^{th} aspect according to any one of the previous aspects, the through channels of the second pair (8a, 8b) of the selective connection body (6) have a substantially rectilinear development.

In a 45^{th} aspect according to the 27^{th} aspect or any one of the previous aspects from the 28^{th} aspect to the 44^{th} aspect when depending on the 27^{th} aspect, the through channels of the second pair (8a, 8b) of the selective connection body (6) have corresponding developments inclined with respect to the walls (6a, 6b, 6c) of the selective connection body (6) of the switching valve (1).

In a 46^{th} aspect according to the 37^{th} aspect or any one of the previous aspects from the 38^{th} aspect to the 45^{th} aspect when depending on the 37^{th} aspect, a fluidic connection end (8a', 8a", 8b', 8b") of each through channel of the second pair (8a, 8b) of the selective connection body (6) of the switching valve (1) is localized along a same generatrix of the second wall (6b) of the selective connection body (6) where a fluidic connection end (7a', 7a", 7b', 7b") of one through channel of the first pair (7a, 7b) of the selective connection body (6) of the switching valve (1) is localized.

In a 47^{th} aspect according to the 37^{th} aspect or any one of the previous aspects from the 38^{th} aspect to the 46^{th} aspect when depending on the 37^{th} aspect, each fluidic connection end (8a', 8a", 8b', 8b") of each through channel of the second pair (8a, 8b) of the selective connection body (6) of the switching valve (1) is localized along a same generatrix of the second wall (6b) of the selective connection body (6) where a corresponding fluidic end (7a', 7a", 7b', 7b") of a respective through channel of the first pair (7a, 7b) of the selective connection body (6) of the switching valve (1) is localized.

In a 48^{th} aspect according to any one of the previous aspects, the through channels of the third pair (9a, 9b) of the selective body (6) of the switching valve (1) develop alongside each other, without intersecting.

In a 49^{th} aspect according to any one of the previous aspects, a same median plane (M), in particular a same radial median plane (M), of the selective connection body (6), of the switching valve (1), is interposed between the through channels of the third pair (9a, 9b) of the selective connection body (6).

In a 50^{th} aspect according to any one of the previous aspects, each through channel of the third pair (9a, 9b) of the selective connection body (6) develop according to broken line.

In a 51^{st} aspect according to any one of the previous aspects, each through channel of the third pair (9a, 9b) of the selective connection body (6) has an elbow course.

In a 52^{nd} aspect according to any one of the previous aspects, each through channel of the third pair (9a, 9b) of the selective connection body (6) has a course having at least one bend (9a'", 9b‴), in particular in the middle.

In a 53^{rd} aspect according to the previous aspect, the bend (9a'", 9b‴) of each through channel of the third pair (9a, 9b) of the selective connection body (6) of the switching valve (1) defines a shape convex towards the other through channel of the third pair (9a, 9b) of the selective connection body (6), and concave on the opposite side.

In a 54^{th} aspect according to according to the 27^{th} aspect or any one of the previous aspects from the 28^{th} aspect to the 53^{rd} aspect when depending on the 27^{th} aspect, the through channels of the third pair (9a, 9b) of the selective connection body (6) of the switching valve (1) have a development inclined with respect to the first wall (6a) and the third wall (6b) of the selective connection body (6).

In a 55^{th} aspect according to any one of the previous aspects, the through channels of the third pair (9a, 9b) of the selective connection body (6) of the switching valve (1) develop at different levels of the latter.

In a 56^{th} aspect according to the 46^{th} aspect or the 47^{th} aspect or any one of the previous aspects from the 48^{th} aspect and the 55^{th} aspect when depending on the 46^{th} aspect and the 47^{th} aspect, a fluidic connection end (9a', 9b') of each through channel of the third pair (9a, 9b) of the selective connection body (6) of the switching valve (1) is localized along a same generatrix of the second wall (6b) of the selective connection body (6) where a fluidic connection end (7a', 7b') of one through channel of the first pair (7a, 7b) of the selective connection body (6) of the switching valve (1) is localized and a fluidic connection end (8a', 8b') of one through channel of the second pair (8a, 8b) of the selective connection body (6) of the switching valve (1) is localized.

In a 57^{th} aspect according to the previous aspect, two generatrix of the second wall (6b) of the selective connection body (6), distinct and separate from each other, are provided with three fluidic connection ends (7a', 7b', 8a', 8b', 9a', 9b'), each corresponding to a specific through channel of the first (7a, 7b), the second (8a, 8b) and the third pair (9a, 9b) of the selective connection body (6) respectively and other two generatrix of the second wall (6b) of the selective connection body (6), distinct and separate from each other and from any other generatrix, are provided with two fluidic connection ends (7a", 7b", 8a", 8b"), each corresponding to a specific through channel of the first (7a, 7b) and the second pair (8a, 8b) of the selective connection body (6) respectively.

In a 58^{th} aspect according to the previous aspect, further two generatrix of the second wall (6b) of the selective connection body (6), distinct and separate from each other and from any other generatrix, are provided with only one fluidic connection end (9a", 9b") of a corresponding through channel of the third pair (9a, 9b) of the selective connection body (6) of the switching valve (1).

in a 59^{th} aspect according to the 6^{th} aspect or any one of the previous aspects from the 7^{th} aspect to the 58^{th} aspect when depending on the 6^{th} aspect, at least an interspace (14) is defined between the housing (10) and the selective connection body (6) of the switching valve (1).

In a 60^{th} aspect according to the 6^{th} aspect or any one of the previous aspects from the 7^{th} aspect to the 59^{th} aspect when depending on the 6^{th} aspect, the selective connection body (6) is smaller than the housing (10) of the switching valve (1) so that to define an interspace (14) between the housing (10) and the selective connection body (6).

In a 61^{st} aspect according to the 6^{th} aspect or any one of the previous aspects from the 7^{th} aspect to the 60^{th} aspect when depending on the 6^{th} aspect, the selective connection body (6) is smaller than the inner chamber (10a) of the housing (10) of the switching valve (1) so that to define an interspace (14) between the housing (10) and the selective connection body (6).

In a 62^{nd} aspect according to the 27^{th} aspect or any one the aspects depending on the 27^{th} aspect when the 27^{th} aspect depends on the aspect 16^{th} or any one of the aspects from the aspect 17^{th} to the aspect 26^{th} when depending on the 17^{th} aspect, the first (6a), the second (6b) and the third wall (6c) of the selective connection body (6) of the switching valve (1) are respectively internally spaced from the first (11), the second (12) and the third wall (13) of the housing (10) of the switching valve (1) to define an interspace (14) between the selective connection body (6) and the housing (10).

In a 63^{rd} aspect according to any one of the four previous aspects at least, depending on the switching condition (i, ii, iii) of the selective connection body (6) of the switching valve (1), the pair of the through channels (7a, 7b, 8a, 8b, 9a, 9b) of the selective connection body (6) which result to be connected to the transit ports (2, 3) and the circuit ports (4, 5) of the housing (10) of the switching valve (1) are fluidically isolated with respect to interspace (14) defined between the selective connection body (6) and the housing (10) of the switching valve (1), the remaining through channels (7a, 7b, 8a, 8b, 9a, 9b) of the other pairs of through channels of the selective connection body (6) not connected to the transit ports (2, 3) and the circuit ports (4, 5) of the housing (10) of the switching valve (1) are in fluid communication with the interspace (14) defined between the selective connection body (6) and the housing (10) of the switching valve (1).

In a 64^{th} aspect according to any one of the five previous aspects, at leas one seal (15) is provided between the housing (10) and the selective connection body (6) of the switching valve (1) at each port (2, 3, 4, 5) of the housing (10) to fluidically isolate the through channels (7a, 7b, 8a, 8b, 9a, 9b) of the selective connection body (6) connected to such ports (2, 3, 4, 5) with respect to the interspace (14) defined between the selective connection body (6) and the housing (10) of the switching valve (1).

In a 65^{th} aspect according to the previous aspect, each seal (15) comprises an annular seal, in particular an O-ring seal, lying into the interspace (14) at the corresponding port (2, 3, 4, 5) of the housing (10) as to surround and isolate such a port (2, 3, 4, 5) with respect to the interspace (14).

In a 66^{th} aspect according any one of the seven previous aspects, the housing (10) of the switching valve (1) is provided with a supply port (16) and a discharge port (17) in fluid communication with the interspace (14) and in fluid communication with a flushing circuit of the extracorporeal blood circuit (100), a flushing fluid arriving from the supply port (16) of the housing (10) and filling the interspace (14) provides the flushing of the through channels (7a, 7b, 8a, 8b, 9a, 9b) of the selective connection body (6) not connected to the transit ports (2, 3) and the circuit ports (4, 5) of the housing (10) to be discharged from the interspace (14) through the discharge port (17) of the housing (10) of the switching valve (1).

In a 67^{th} aspect according to the previous aspect, the discharge port (17) and the supply port (16) of the housing (10) of the switching valve (1) are respectively localized in correspondence of the first wall (11) and the third wall (13) of such a housing (10).

In a 68^{th} aspect according to any one of the previous aspects the switching valve (1) comprises four ports (2, 3, 4, 5) and six through channels (7a, 7b, 8a, 8b, 9a, 9b), in particular each through channel (7a, 7b, 8a, 8b, 9a, 9b) being distinct and separate with respect to any other through channel (7a, 7b, 8a, 8b, 9a, 9b).

In a 68^{th} bis aspect according to any one of the previous aspects, the treatment unit (200) comprises (or is) a filtration unit (200a) to filter blood, the filtration unit (200a) being for example an adsorber, a plasma filter, an apheresis filter or a unit for treating blood of patient with renal failure, such as an ultrafilter, a hemofilter, a hemodiafilter, a hemodialysis filter.

In a 68^{th} ter aspect according to any one of the previous aspects, the treatment unit (200) comprises (or is) a gas exchanger for e.g., removing CO₂ from the extracorporeal blood.

In a 69^{th} aspect according to any one of the previous aspects, the treatment unit (200) has a primary chamber (203) and a secondary chamber (204) separated by a semi-permeable membrane (205), the inlet (201) of the treatment unit (200) connecting the blood withdrawal line (301) of the extracorporeal blood circuit (300) to the primary chamber (203) of the treatment unit (200), the outlet (202) of the treatment unit (200) connecting the blood return line (302) of the extracorporeal blood circuit (300) to primary chamber (204) of the treatment unit (200).

In a 69^{th} bis aspect according to any one the previous aspects, the treatment unit (200) has a blood side and an air side separated by a gas-permeable membrane, the inlet (201) of the treatment unit (200) connecting the blood withdrawal line (301) of the extracorporeal blood circuit (300) to the blood side of the treatment unit (200), the outlet (202) of the treatment unit (200) connecting the blood return line (302) of the extracorporeal blood circuit (300) to the blood side of the treatment unit (200).

In a 70th aspect according to the previous aspect 69, the extracorporeal blood treatment apparatus (100) comprises: a dialysate line (400) connected to the secondary chamber (204) of the treatment unit (200) by means of an auxiliary outlet (207) of the treatment unit (200) and provided with a dialysate pump (401); and, optionally, a supply line (402) connected to the secondary chamber (204) of the treatment unit (200) by means of an auxiliary inlet (206) of the treatment unit (200) and provided with a supply pump (403).

In a 71^{st} aspect according to any one of the previous aspects, the extracorporeal blood treatment apparatus (100) comprises: a first infusion line (500) connected to the blood withdrawal line (301) of the extracorporeal blood circuit (300) to transfer a first infusion solution (501) into blood; a first infusion fluid source (502) of the first infusion solution (501) connected to the first infusion line (500); a first infusion pump (503) active on the first infusion line (500).

In a 72^{nd} aspect according to the previous aspect, the first infusion fluid source (502) comprises at least one bag, optionally made of a plastic material, for example the bag of the first infusion fluid source (502) is flexible and made of two opposite leaves joined, optionally hermetically sealed, each other, in particular welded each other.

In a 73^{rd} aspect according to any one of the two previous aspects, the first infusion line (500) is connected to the blood withdrawal line (301) upstream of a blood pump (101) of the extracorporeal blood circuit (300).

In a 74^{th} aspect according to any one of the three previous aspects, the bag of the first infusion fluid source (502) is disposable.

In a 76^{th} aspect according to any one of the previous aspects, the extracorporeal blood treatment apparatus (100) comprises: a second infusion line (600) connected to the blood withdrawal line (301) of the extracorporeal blood circuit (300) to transfer a second infusion solution (601) into blood; a second infusion fluid source (602) of the second infusion solution (601) connected to the second infusion line (600); a second infusion pump (603) active on the second infusion line (600).

In a 77^{th} aspect according to the previous aspect, the second infusion fluid source (602) comprises at least one bag, optionally made of a plastic material.

In a 78^{th} aspect according to any one of the two previous aspects, the bag of the second infusion fluid source (602) is flexible and made of two opposite leaves joined, optionally hermetically sealed, each other, in particular welded each other.

In a 79^{th} aspect according to any one of the three previous aspects, the bag of the second infusion fluid source (602) is disposable.

In a 80^{th} aspect according to any one of the previous aspects, the extracorporeal blood treatment apparatus (100) comprises a blood pump (101) active on the extracorporeal blood circuit (300).

In a 81^{st} aspect according to the previous aspect, the blood pump (101) is placed on the withdrawal line (301) of the extracorporeal blood circuit (300).

In a 82^{nd} aspect according to the 71^{st} aspect or any one of the nine previous aspects when depending on the 71^{st} aspect, the blood pump (101) is placed between the treatment unit (200) and a first injection point (504) of the first infusion line (500) which is placed on the blood withdrawal line (301).

In a 83^{rd} aspect according to the 76^{th} aspect or any one of the six previous aspects when depending on the 76^{th} aspect, a second injection point (604) of the second infusion line (600) is placed on the blood withdrawal line (301) between the treatment unit (200) and the blood pump (101).

In a 84^{th} aspect according to the 76^{th} aspect or any one of the seven previous aspects when depending on the 76^{th} aspect, the second infusion line (600) is also connected to the supply line (402) by means of a first branch (605) which develops from a first forking point (606) placed on the second infusion line (600) between the second infusion pump (603) and the blood withdrawal line (301) of the extracorporeal blood circuit (300) and a third injection point (607) placed on the supply line (402) between the supply pump (403) and the treatment unit (200).

In a 85^{th} aspect according to the 77^{th} aspect, the second infusion line (600) is also connected to the blood return line (302) of the extracorporeal al blood circuit (300) by means of a second branch (608) developing from a second forking point (609) placed on the first branch (605) of the second infusion line (600) and a fourth injection point (610) placed on the blood return line (302).

In a 86^{th} aspect according to any one of the two previous aspects, the second infusion line (600) comprises a first selective device (611) operatively placed between the first forking point (606) and the blood withdrawal line (301) of the extracorporeal blood circuit (300), in particular close to or at the first forking point (606), the first selective device (611) being drivable between a first condition, wherein the second infusion solution (601) arriving from the second infusion fluid source (602) can flow only along the second infusion line (600) towards the blood withdrawal line (301) of the extracorporeal blood circuit (300), and a second condition wherein the second infusion solution (601) arriving from the second infusion fluid source (602) can flow only along the first branch (605) of the second infusion line (600) towards the supply line (402).

In a 86^{th} aspect according to any one of the three previous aspects, a second selective device (612) is operatively placed in correspondence of the fourth injection point (610) of the first branch (605) of the second infusion line (600), the second selective device (612) being drivable between a first condition wherein the second infusion solution (601) flowing along the first branch (605) of the second infusion line (600) is free to flow into the supply line (402), and a second condition wherein the second infusion solution (601) flowing along the first branch (605) of the second infusion line (601) can only flow along the second branch (608) of the second infusion line (600) towards the blood return line (302) of the extracorporeal blood circuit (300).

In a 87^{th} aspect according to any one of the previous aspects, a control unit (18) is operatively connected to the switching valve (1) and is configured to drive the switching valve (1) between the first switching condition (i), the second switching condition (ii) and the third switching condition (iii), to respectively trigger the normal mode of operation, the reversing mode of operation and the recirculation mode.

In a 88^{th} aspect according to the previous aspect when depending on the 1^{st} ter independent aspect or any one of the aspects from the 1^{st} quarter independent aspect to the 86^{th} aspect when depending on the 1^{st} ter independent aspect, the control unit (18) is configured to rotationally drive the selective connection body (6) of the switching valve (1) between the first switching condition (i), the second switching condition (ii) and the third switching condition (iii), to respectively trigger the normal mode of operation, the reversing mode of operation and the recirculation mode.

In a 89^{th} aspect according to the any one of the two previous aspects when the 87^{th} aspect depends on the 30^{th} aspect or any one of the previous aspects depending on the 30^{th} aspect, the control unit (18) is connected to the actuator arranged to move, in particular to rotate, the transmission shaft (6d) of the selective connection body (6) of the switching valve (1), the control unit (18) being configured to drive the actuator to move the selective connection body (6) between the first switching condition (i), the second switching condition (ii) and the third switching condition (iii), to respectively trigger the normal mode of operation, the reversing mode of operation and the recirculation mode.

In a 90^{th} aspect according to the any one of the previous aspects, wherein the switching valve (1) is configured to be rotated (e.g., manually or automatically) in order to be configured between the first switching condition (i), the second switching condition (ii) and the third switching condition (iii), to respectively trigger the normal mode of operation, the reversing mode of operation and the recirculation mode of operation.

Additionally, any of the above aspects, or portions thereof, and/or any of the features, functionality and alternatives described in connection with any one or more of attached Figures may be combined with any of the features, functionality and alternatives described in connection with any other of above aspects, what is described and shown in the Figures.

Additional features and advantages are described in, and will be apparent from, the following detailed description and the Figures. The features and advantages described herein are not all-inclusive and, in particular, many additional features and advantages will be apparent to one of ordinary skill in the art in view of the figures and description. Also, any particular embodiment does not have to have all of the advantages listed herein and it is expressly contemplated to claim individual advantageous embodiments separately. Moreover, it should be noted that the language used in the specification has been selected principally for readability and instructional purposes, and not to limit the scope of the inventive subject matter.

### BRIEF DESCRIPTION OF THE FIGURES

Figure 1 is a schematic view of a fluid circuit of an extracorporeal blood treatment apparatus according to a first embodiment of the present disclosure, schematically represented in a given operating condition corresponding to a normal mode of operation and with two versions a switching valve block;
Figure 2 is a schematic view of the fluid circuit of the extracorporeal blood treatment apparatus shown in Figure 1, schematically represented in another given operating condition corresponding to a reverse mode of operation different to the condition shown in Figure 1 and with two versions of the switching valve block;
Figure 3 is a schematic view of the fluid circuit of the extracorporeal blood treatment apparatus shown in Figures 1 and 2, schematically represented in a further given operating condition corresponding to a recirculation mode of operation different to the conditions shown in Figures 1 and 2 and with two versions of the switching valve block;
Figure 4 is a schematic view of a fluid circuit of an extracorporeal blood treatment apparatus according to a second embodiment of the present disclosure;
Figure 5 is a perspective view of the switching valve of the extracorporeal blood circuit of the extracorporeal blood treatment apparatus schematically shown in Figures 1 to 4;
Figure 6 is a median cross-section of the switching valve executed along the trace VI-VI of Figure 5;
Figure 7 is a perspective view of a selective connection body of the switching valve shown in Figures 5 and 6;
Figure 8 is an elevation view of the selective connection body of the switching valve shown in figures 5 to 8;
Figure 9 is a top-down view of the selective connection body of the switching valve shown in figures 5 to 9.

### DETAILED DESCRIPTION

### Extracorporeal blood treatment apparatus and extracorporeal blood circuit

Extracorporeal blood treatment (for example, but not exclusively, renal failure dialysis) may be used in patients with rapidly developing loss of kidney function, called acute renal failure or slowly worsening kidney function, called Stage 5 chronic kidney disease (or end-stage renal disease). In the following description, some embodiments of extracorporeal blood treatment apparatuses will be firstly described being suitable, or designed, principally (though not exclusively) for intensive care treatments.

Unless defined otherwise, all technical and scientific terms used have the same meaning as commonly understood by one of ordinary skill in the art.

With reference to the attached figures, the numeral 100 globally refers to an extracorporeal blood treatment apparatus which may be used for performing any kind of kidney failure therapy, such as Hemodialysis ("HD"), Hemofiltration ("HF"), Hemodiafiltration ("HDF"), Hemoperfusion and in the context of continuous renal replacement therapies (CRRT) with or without anticoagulation, for example CRRT with or without systemic anticoagulation (e.g., heparin) / with or without regional anticoagulation (e.g., citrate).

The extracorporeal blood treatment apparatus 100 comprises a treatment unit 200 and an extracorporeal blood circuit 300. The extracorporeal blood circuit 300 comprises a blood withdrawal line 301 connected to an inlet 201 of the treatment unit 200 and connectable to a first access port A or a second access port B to a patient P, opposite to the treatment unit 200, for the passage of blood inwards the extracorporeal blood circuit 300, namely from the patient P to the extracorporeal blood circuit 300. The extracorporeal blood circuit 300 also comprises a blood return line 302 connected to an outlet 202 of the treatment unit 200 and connectable to the second access port B or the first access port A to the patient P, opposite to the treatment unit 200, for the passage of blood outwards the extracorporeal blood circuit 300, namely from the extracorporeal blood circuit 300 to the patient P.

The treatment unit 200 comprises or is a filtration unit 200a configured to filter blood. For example, the treatment unit 200 comprises an adsorber or a plasma filter or an apheresis filter or a unit for treating blood of patient with renal failure, such as an ultrafilter, a hemofilter, a hemodiafilter, a hemodialysis filter.

The treatment unit 200 can also comprises or can be a gas exchanger for removing CO2 from the extracorporeal blood.

The treatment unit 200 has a primary chamber 203 and a secondary chamber 204 separated by a semi-permeable membrane 205. The inlet 201 of the treatment unit 200 connects the blood withdrawal line 301 of the extracorporeal blood circuit 300 to the primary chamber 203 of the treatment unit 200, while the outlet 202 of the treatment unit 200 connects the blood return line 302 of the extracorporeal blood circuit 300 to primary chamber 204 of the treatment unit 200.

The treatment unit 200 has also a blood side and an air side separated by a gas-permeable membrane. Both the withdrawal line 301 and the return line 302 of the extracorporeal blood circuit 300 are connected to the blood side of the treatment unit 200, in particular respectively to the inlet 201 and the outlet 202 of the treatment unit 200.

The extracorporeal blood treatment apparatus 100 is provided with a blood pump 101 which is preferably active on the withdrawal line 301 upstream of the treatment unit 200.

The extracorporeal blood treatment apparatus 100 also comprises a dialysate line 400 which is connected to the secondary chamber 204 of the treatment unit 200 by means of an auxiliary outlet 207 of the treatment unit 200 and is provided with a dialysate pump 401. Optionally, the extracorporeal blood treatment apparatus 100 comprises a supply line 402 which is connected to the secondary chamber 204 of the treatment unit 200 through an auxiliary inlet 206 of the treatment unit 200 and is provided with a supply pump 403.

The extracorporeal blood treatment apparatus 100 further comprises a first infusion line 500 which is connected, through a first injection point 504, to the blood withdrawal line 301 of the extracorporeal blood circuit 300 to transfer a first infusion solution 501 into blood. The first infusion solution 501 is supplied by a first infusion fluid source 502 which is connected to the first infusion line 500. A first infusion pump 503, active on the first infusion line 500, ensures the first infusion solution 501 to flow from the first infusion fluid source 502 to the blood withdrawal line 301 of the extracorporeal blood circuit 300. The blood pump 101 is placed between the treatment unit 200 and the first injection point 504 of the first infusion line 500.

The first infusion fluid source 502 can comprise at least one bag, optionally made of a plastic material. For example, the bag of the first infusion fluid source 502 is flexible and made of two opposite leaves joined, optionally hermetically sealed, each other, in particular welded each other. The bag of the first infusion fluid source 502 can be disposable.

According to an embodiment of the present disclosure, the first infusion line 500 is connected, or connectable, to the blood withdrawal line 301 upstream of the blood pump 101 of the extracorporeal blood circuit 300.

The extracorporeal blood treatment apparatus 100 also comprises a second infusion line 600 connected, or connectable, through a second injection point 604, to the blood withdrawal line 301 of the extracorporeal blood circuit 300 to transfer a second infusion solution 601 into blood. The second injection point 604 of the second infusion line 600 is placed on the blood withdrawal line 301 between the treatment unit 200 and the blood pump 101. The second infusion solution 601 is supplied by a second infusion fluid source 602 which is connected to the second infusion line 600. A second infusion pump 603, active on the second infusion line 600, ensures the second infusion solution 601 to flow from the second infusion fluid source 602 to the blood withdrawal line 301 of the extracorporeal blood circuit 300.

Also the second infusion fluid source 602 can comprise at least one bag, optionally made of a plastic material. Such a bag is flexible and made of two opposite leaves joined, optionally hermetically sealed, each other, in particular welded each other. Also the bag of the second infusion fluid source 602 can be disposable.

According to another embodiment, the second infusion line 600 is connected to the supply line 402 by means of a first branch 605 which develops from a first forking point 606 placed on the second infusion line 600 between the second infusion pump 603 and the blood withdrawal line 301 of the extracorporeal blood circuit 300 and a third injection point 607 placed on the supply line 402 between the supply pump 403 and the treatment unit 200. The second infusion line 600 is also connected to the blood return line 302 of the extracorporeal al blood circuit 300 by means of a second branch 608 developing from a second forking point 609 placed on the first branch 605 of the second infusion line 600 and a fourth injection point 610 placed on the blood return line 302.

The second infusion line 600 comprises a first selective device 611 operatively placed between the first forking point 606 and the blood withdrawal line 301 of the extracorporeal blood circuit 300, in particular close to or at the first forking point 606. The first selective device 611 is drivable between a first condition, wherein the second infusion solution 601 arriving from the second infusion fluid source 602 can flow only along the second infusion line 600 towards the blood withdrawal line 301 of the extracorporeal blood circuit 300, and a second condition wherein the second infusion solution 601 arriving from the second infusion fluid source 602 can flow only along the first branch 605 of the second infusion line 600 towards the supply line 402.

A second selective device 612 is operatively placed in correspondence of the fourth injection point 610 of the first branch 605 of the second infusion line 600. The second selective device 612 is drivable between a first condition wherein the second infusion solution 601 flowing along the first branch 605 of the second infusion line 600 is free to flow into the supply line 402, and a second condition wherein the second infusion solution 601 flowing along the first branch 605 of the second infusion line 601 can only flow along the second branch 608 of the second infusion line 600 towards the blood return line 302 of the extracorporeal blood circuit 300.

### Switching valve

Advantageously, the extracorporeal blood treatment apparatus 100 comprises at least one switching valve 1, in particular substantially cylindrical, in fluid communication with the blood withdrawal line 301 and the blood return line 302 of the extracorporeal blood circuit 300 and in fluid communication with the first access port A and the second access port B at the patient P.

The switching valve 1 is switchable between three switching conditions, namely a first, a second and a third switching condition.

In the first switching condition, the first access port A and the second access port B are respectively fluidly connected to the blood withdrawal line 301 of the extracorporeal blood circuit 300 and the blood return line 302 of the extracorporeal blood circuit 300 to trigger a normal mode of operation of the extracorporeal blood circuit 300 wherein blood coming from the first access port A flows through the switching valve 1, the withdrawal line 301 of the extracorporeal blood circuit 300, the treatment unit 200, the blood return line 302 of the extracorporeal blood circuit 300, the switching valve 1 again, to arrive at the second access port B.

In the second switching condition, the first access port A and the second access port B are respectively fluidly connected to the blood return line 302 of the extracorporeal blood circuit 300 and the blood withdrawal line 301 of the extracorporeal blood circuit 300 to trigger a reversing mode of operation of the extracorporeal blood circuit 300 wherein blood coming from the second access port B flows through the switching valve 1, the blood withdrawal line 301 of the extracorporeal blood circuit 300, the treatment unit 200, the blood return line 302 of the extracorporeal blood circuit 300, the switching valve 1 again, to arrive to the first access port A.

In the third switching condition, the first access port A and the second access port B are fluidly connected to each other and the blood withdrawal line 301 and the blood return blood line 302 of the extracorporeal blood circuit 6 are fluidly connected to each other to trigger a recirculation mode of the extracorporeal blood circuit 300, wherein the extracorporeal blood circuit 300 is not in fluid communication with the first A and the second access port B.

In order to perform the three switching conditions above mentioned, the switching valve 1 has a specific structure which will be described in the following.

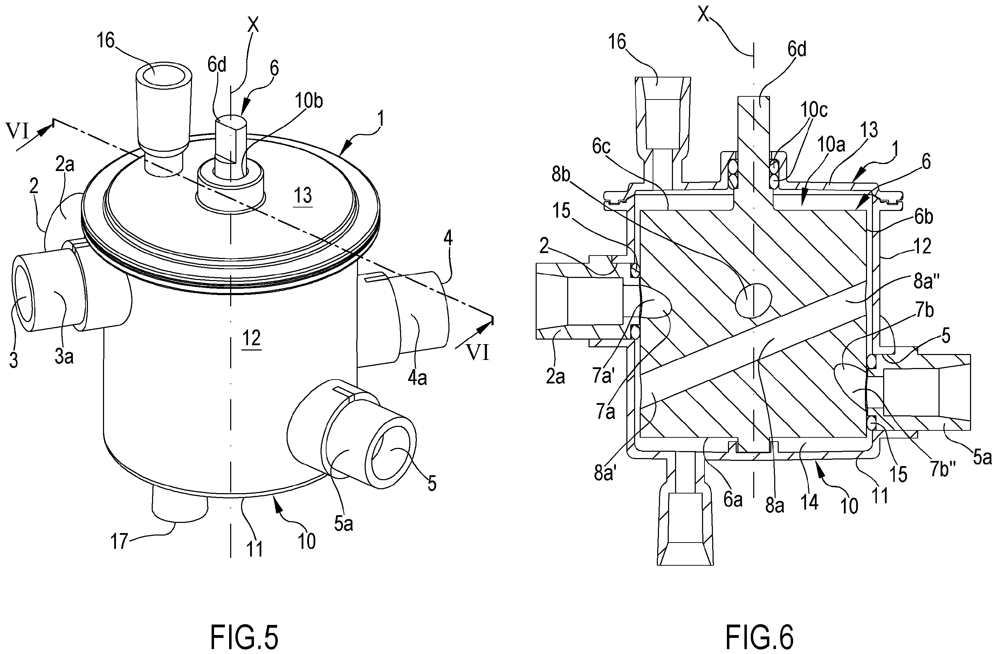

According to the embodiment shown in Figures 5 and 6, the switching valve 1 comprises a housing 10 defining an inner chamber 10a and a selective connection body 6 which operatively, in particular rotationally, occupies the inner chamber 10a of the housing 10 to determine the specific switching condition in relation to the mode of operation to be triggered.

The housing 10 of the switching valve 1 is substantially cylindrical so that both the inner chamber 10a and the selective connection body 6 are cylindrical too.

In particular, the housing 10 of the switching valve 1 is defined by or between a first substantially circular wall 11, a second substantially cylindrical wall 12, transversally developing from the first circular wall 11, and a third substantially circular wall 13 transversally engaged to the second wall 12, on the opposite side to the first wall 11. The first circular wall 11 defines the bottom portion of the housing 10, the second cylindrical wall 12 defines the lateral shell of the housing 10, while the third circular wall 13 defines the top portion of the housing 10.

As in Figures 5 to 9, the selective connection body 6 of the switching valve 1 comprises a first substantially circular wall 6a, a second substantially cylindrical wall 6b, transversally developing from the first circular wall 6a, and a third substantially circular wall 6c, transversally developing from the second wall 6b and facing away from the first wall 6a. The first circular wall 6a defines the bottom portion of the selective connection body 6, the second cylindrical wall 6b defines the lateral wall of the selective connection body 6, while the third circular wall 6c defines the top portion of the selective connection body 6.

The first circular wall 6a, the second cylindrical wall 6b and the third circular wall 6c of the selective connection body 6 of the switching valve 1 respectively face internally at the first circular wall 1, the second cylindrical wall 12 and the third circular wall 13 of the housing 10 of the switching valve 1.

As in Figures 5 to 9, the selective connection body 6 of the switching valve 1 is provided with at least one transmission shaft 6d engageable, directly or indirectly, to an actuator device able to move the transmission shaft 6d and the selective connection body 6 between the three switching conditions of the switching valve 1 so that to trigger the corresponding operational modes. The transmission shaft 6d protrudes from the selective connection body 6, in particular developing from the third circular wall 6c of the selective connection body 6 and along a longitudinal axis X of the selective connection body 6 through an opening 10b cut out in the center of the third circular wall 13 of the housing 10.

At least one seal 10c, in particular two seals 10c superimposed, e.g. O-ring seals, are interposed between the third wall 13 of the housing 10 of the switching valve 1 and the transmission shaft 6d of the switching valve 1 in order to avoid that any fluid leaks from the inner chamber 10a of the housing 10 and/or from the outside towards the inner chamber 10a of the housing along the transmission shaft 6d.

The switching valve 1 is provided with a first transit port 2 in fluid communication with the first access port A, a second transit port 3 in fluid communication with the second access port B, a first circuit port 4 in fluid communication with the blood withdrawal line 301 of the extracorporeal blood circuit 300, a second circuit port 6 in fluid communication with the blood return line 302 of the extracorporeal blood circuit 300. Different pairs of ports 2, 3, 4, 5 of the switching valve are connected together by the selective connection body 6. The angular position of the selective connection body 6 of the switching valve 1 determines what pair of ports 2, 3, 4, 5, are connected together.

All ports 2, 3, 4, 5, are defined on, and/or through, the housing 10, in particular on, and/or through, the second cylindrical wall 12 of the housing 10. Each port 2, 3, 4, 5, is provided with a corresponding tubular fitting 2a, 3a, 4a, 5a, protruding externally from the second cylindrical wall 12 of the housing 10 for the connection with an access port A, B to the patient P or a line 301, 302 of the extracorporeal blood circuit 300.

As shown in Figure 5, the first and the second transit ports 2, 3 of the switching valve 1 are positioned at different levels or lying planes with respect to the levels or lying plains of the first and the second circuit ports 4, 5 of the switching valve 1. Each port 2, 3, 4, 5, is positioned at a corresponding level or lying plane different with respect to the levels or lying planes of any one of the other ports 2, 3, 4, 5.

The first 2 and the second transit port 3 are positioned close to the third circular wall 13 of the housing 10 of the switching valve 1, while the first 4 and the second circuit port 5 are positioned close to the first wall 11 of the housing 10 of the switching valve 1.

Furthermore, at least one of the first and second transit port 2, 3 of the switching valve 1 has a corresponding longitudinal development axis lying on a radial plane of the switching valve 1 on which a longitudinal development axis of at least one of the first and the second circuit port 4, 5 lies. In particular, ach of the transit ports 2, 3 has a corresponding longitudinal axis lying on a radial plane of the switching valve 1 on which a longitudinal development axis of a corresponding circuit port 4, 5 of the switching valve 1 lies.

According to the embodiments shown in Figures 5 and 6, the first and the second transit ports 2, 3 of diverge from each other away from the second cylindrical wall 12 of the housing 10 so that also the corresponding tubular fittings 2a, 3a diverge from each other, away from the housing 10 of the switching valve 1. Similarly, the first and the second circuit ports 4, 5 diverge from each other away from the second cylindrical wall 12 of the housing 10 so that the corresponding tubular fittings 4a, 5a diverge from each other, away from the housing 10 of the switching valve 1.

The transit ports 2, 3 of the switching valve 1 are positioned at an angle to each other comprised between 10° and 100°, in particular comprised between 20° and 90°, more in particular comprised between 30° ad 80°, optionally comprised between 50° and 70°, more optionally around 60°. It is the same for the circuit ports 4, 5. They are positioned at an angle to each other comprised between 10° and 100°, in particular comprised between 20° and 90°, more in particular comprised between 30° ad 80°, optionally comprised between 50° and 70°, more optionally around 60°.

In order to ensure different connections between different combinations of pairs of ports 2, 3, 4, 5, the selective connection body 6 is provided with six through channels 7a, 7b, 8a, 8b, 9a, 9b, each of them has a circular cross-section profile. A first pair 7a, 7b of through channels is configured to respectively fluidly connect the first transit port 2 with the first circuit port 4 and the second transit port 3 with the second circuit port 5 of the switching valve 1. a second pair 8a, 8b of through channels, distinct with respect to the through channels of the first pair 7a, 7b atre configured to respectively fluidly connect the first transit port 2 with the second circuit port 5 and the second transit port 3 with the first circuit port 4. A third pair 9a, 9b of through channels distinct with respect to the through channels of the first 7a, 7b and the second pairs 8a, 8b are configured respectively to fluidly connect the first transit port 2 to the second transit port 3 of the switching and the first circuit port 4 with the second circuit port 5.

The through channels 7a, 7b, 8a, 8b, 9a, 9b of the selective connection body 6 of the switching valve 1 and/or the ports 2, 3, 4, 5 of the housing 10 of the switching valve 1 have a transversal/diametrical size close to, in particular equal to, the transversal/diametrical size of the lines 301, 302 and/or the tubes connecting the transit ports 2, 3 of the housing 10 of the switching valve 1 with the access ports A, B to the patient P, in order to minimize pressure drops.

Each through channel 7a, 7b, 8a, 8b, 9a, 9b of the selective connection body 6 comprises two fluidic connection ends 7a', 7a", 7b', 7b", 8a', 8a", 8b', 8b", 9a', 9a", 9b', 9b" localized in correspondence of the second cylindrical wall 6b to provide a fluid communication with a corresponding pair of ports 2, 3, 4, 5 of the housing 10. A fluidic connection end 7a', 7b', 8a', 8b', 9a', 9b' defines an inlet for a fluid, while the other fluidic connection end 7a", 7b", 8a", 8b", 9a", 9b" defining the outlet for the same fluid. Obviously, the inlet and outlet can be inverted in relation of the specific connection performed, and they are defined by the direction of the fluid into the corresponding through channel 7a, 7b, 8a, 8b, 9a, 9b.

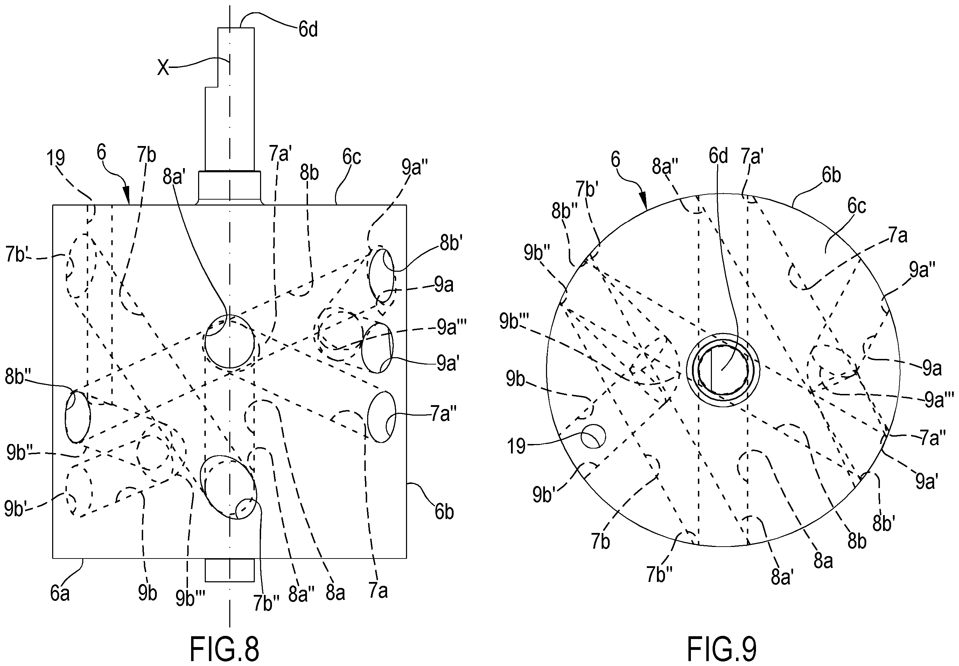

According to the embodiment shown in Figure 9, the through channels of the first pair 7a, 7b have a substantially rectilinear development and develop alongside each other, without intersecting. Therefore, the through channels of the first pair 7a, 7b are spaced out from each other so that a radial median plane M of the selective connection body 6 of the switching valve 1 is interposed between the through channels of the first pair 7a, 7b. In particular, the through channels of the first pair 7a, 7b have a development inclined with respect to the first circular wall 6a and the third circular wall 6c of the selective connection body 6.

The through channels of the second pair 8a, 8b cross a same radial median plane M of the selective connection body 6 of the switching valve 1 and develop rectilinearly and obliquely at different levels of the latter according to respective crossing directions. A fluidic connection end 8a', 8a", 8b', 8b" of each trough channel of the second pair 8a, 8b is localized along a same generatrix of the second cylindrical wall 6b of the selective connection body 6 where a fluidic connection end 7a', 7a", 7b', 7b" of a through channel of the first pair 7a, 7b is localized. They are vertically aligned.

The through channels of the third pair 9a, 9b develop alongside each other, without intersecting. Therefore, the through channels of the third pair 9a, 9b are spaced out from each other so that a same radial median plane M of the selective connection body 6 of the switching valve 1 is interposed between the through channels of the third pair 9a, 9b. In particular, the through channels of the third pair 9a, 9b have a development inclined with respect to the first circular wall 6a and the third circular wall 6b and they develop at different levels of the selective connection body 6.

In order to reverse the direction of the fluid flowing within itself, each through channel of the third pair 9a, 9b develop according to broken line, optionally presenting an elbow course. In other words, each through channel of the third pair 9a, 9b has a course having at least one bend 9a'", 9b'", in particular in the middle. The bend 9a'", 9b‴ of each through channel of the third pair 9a, 9b defines a shape convex towards the other corresponding through channel of the third pair 9a, 9b and concave on the opposite side, namely towards the outside of the selective connection body 6.

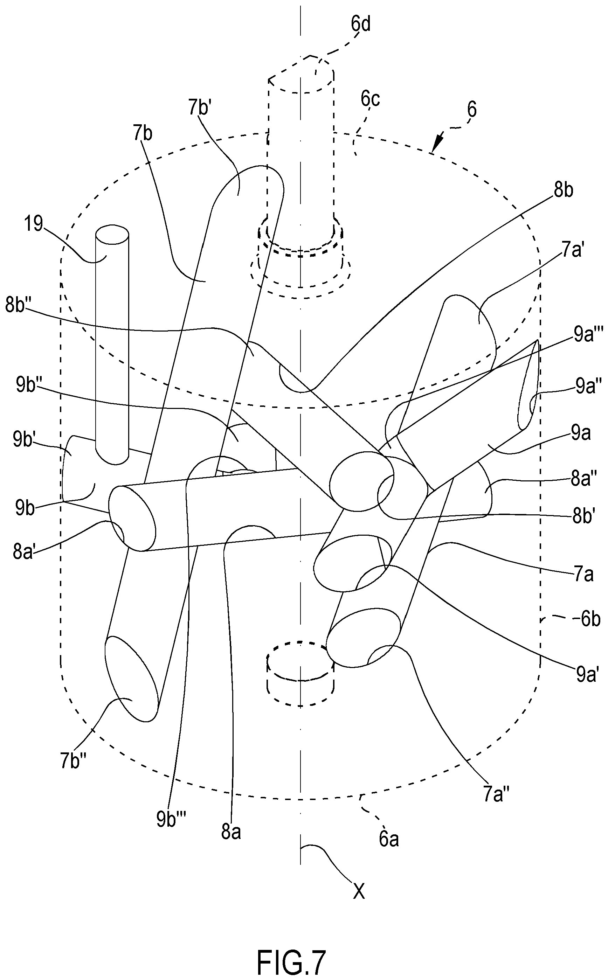

A fluidic connection end 9a', 9b' of each through channel of the third pair 9a, 9b is localized along a same generatrix of the second cylindrical wall 6b of the selective connection body 6 where a fluidic connection end 7a', 7b' of a through channel of the first pair 7a, 7b is localized and a fluidic connection end 8a', 8b' of one through channel of the second pair 8a, 8b of the selective connection body 6 of the switching valve 1 is localized. They are vertically aligned.According to the embodiment shown in Figure 7 and 8, two generatrix of the second cylindrical wall 6b of the selective connection body 6, distinct and separate from each other, are provided with three fluidic connection ends 7a', 7b', 8a', 8b', 9a', 9b', properly and vertically aligned, each corresponding to a specific through channel of the first 7a, 7b, the second 8a, 8b and the third pair 9a, 9b of through channels of the selective connection body 6 respectively and other two generatrix of the second cylindrical wall 6b of the selective connection body 6, distinct and separate from each other and from any other generatrix, are provided with two fluidic connection ends 7a", 7b", 8a", 8b", properly vertically and aligned, each corresponding to a specific through channel of the first 7a, 7b and the second pair 8a, 8b of the through channels of the selective connection body 6 respectively.

Moreover, further two generatrix of the second cylindrical wall 6b of the selective connection body 6, distinct and separate from each other and from any other generatrix, are provided with only one fluidic connection end 9a", 9b" of a corresponding through channel of the third pair 9a, 9b of the selective connection body 6 of the switching valve 1.

As shown in Figure 6, at least an interspace 14 is defined between the housing 10 and the selective connection body 6 of the switching valve 1. In order to define the interspace 14 of the switching valve 1, the selective connection body 6 is smaller than the inner chamber 10a of the housing 10.

Mode in detail, the first circular wall 6a, the second cylindrical wall 6b and the third circular wall 6c of the selective connection body 6 of the switching valve 1 are respectively internally spaced from the first circular wall 11, the second cylindrical wall 12 and the third circular wall 13 of the housing 10 of the switching valve to define an interspace 14 between the selective connection body 6 and the housing 10.

Depending on the switching condition of the selective connection body 6 of the switching valve 1, the pair of the through channels 7a, 7b, 8a, 8b, 9a, 9b of the selective connection body 6 which result to be connected to the transit ports 2, 3 and the circuit ports 4, 5 of the housing 10 of the switching valve 1 are fluidically isolated with respect to interspace 14 defined between the selective connection body 6 and the housing 10 of the switching valve 1, while the remaining through channels 7a, 7b, 8a, 8b, 9a, 9b of the other pairs of through channels of the selective connection body 6 not connected to the transit ports 2, 3 and the circuit ports 4, 5 of the housing 10 of the switching valve 1 are in fluid communication with the interspace 14 defined between the selective connection body 6 and the housing 10 of the switching valve 1. In order to maintain the pair of through channels 7a, 7b, 8a, 8b, 9a, 9b of the selective connection body 6 which result to be connected to the transit ports 2, 3 and the circuit ports 4, 5 of the housing 10 of the switching valve 1 fluidically isolated from the interspace 14, at least one seal 15 is provided between the second cylindrical wall 12 of the housing 10 and the second cylindrical wall 12 of the selective connection body 6 at each port 2, 3, 4, 5 of the housing 10. Each seal 15 comprises an annular seal, in particular an O-ring seal, lying into the interspace 14 at the corresponding port 2, 3, 4, 5 of the housing 10 as to surround and isolate, from the inside of the inner chamber 10a of the housing 10, such a port 2, 3, 4, 5 with respect to the interspace 14.

As it can be seen in Figures 5 and 6, the housing 10 of the switching valve 1 is provided with a supply port 16 and a discharge port 17 in fluid communication with the interspace 14 and in fluid communication with a flushing circuit of the extracorporeal blood circuit 100. The flushing fluid arrives from the supply port 16 of the housing 10 and fills the interspace 14 providing the flushing of the through channels 7a, 7b, 8a, 8b, 9a, 9b of the selective connection body 6 not connected to the transit ports 2, 3 and the circuit ports 4, 5 of the housing 10 to be discharged from the interspace 14 through the discharge port 17 of the housing 10 of the switching valve 1. The discharge port 17 and the supply port 16 of the housing 10 of the switching valve 1 are respectively localized in correspondence of the first wall 11 of and the third wall 13 of such a housing 10.

As shown in Figures 7 to 9, at least one of the through channels of the third pair 9a, 9b of channels of the selective connection body 6 of the switching valve 1 is provided with an additional channel 19, in particular on the through channel 9b. However, it is also possible that the additional channel 19 be provided only on the through channel 9a of the selective connection body 6 of the switching valve 1 or even more on both through channels of the third pair 9a, 9b of channels.

Any possible additional channel 19 vertically develops from the corresponding through channel of the third pair 9a, 9b of channels substantially parallel to the longitudinal axis X of the selective connection body 6 of the switching valve 1 to end at the third wall 6c of the latter where the additional channel 19 is in fluid communication with the interspace 14.

### Switching valve operation

Regarding the operation of the switching valve 1, as already said above, the selective connection body 6 is rotationally movable inside the inner chamber 10a of the housing 10 between three switching conditions.

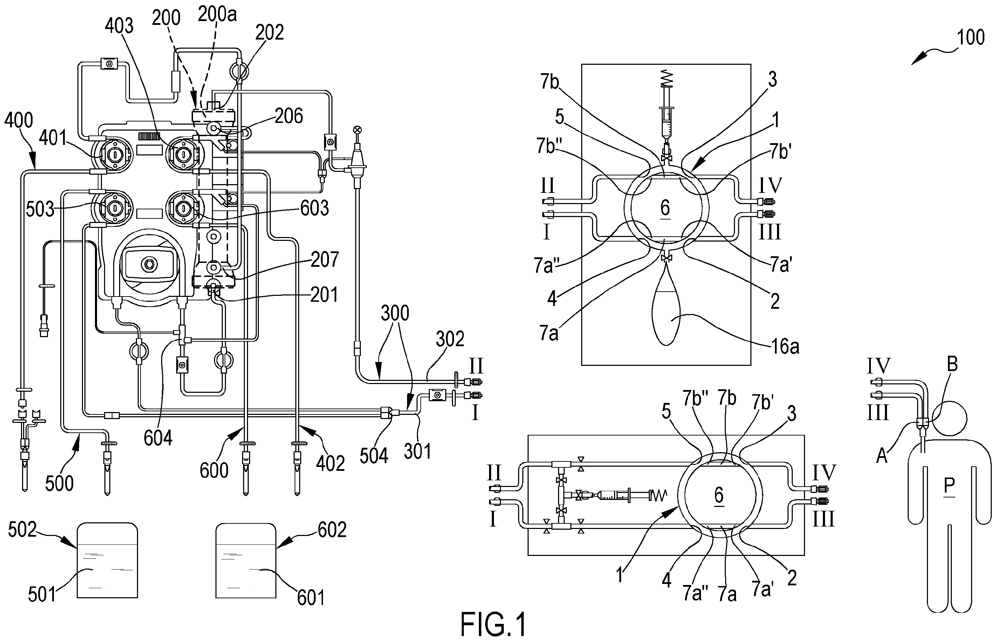

In the first switching condition (Figure 1), the first transit port 2 of the housing of the switching valve 1 is fluidly connected to the first circuit port 4 of the housing 10 of switching valve 1 to put in fluid communication the first access port A with the blood withdrawal line 301 of the extracorporeal blood circuit 300, and the second transit port 3 of housing 10 of the switching valve 1 is fluidly connected to the second circuit port 5 of the housing 10 of the switching valve 1 to put in fluid communication the second access port B with the blood return line 302 of the extracorporeal blood circuit 300. In particular, the first pair 7a, 7b of through channels of the selective connection body 6 of the switching valve 1 fluidly connects the first transit port 2 and the second transit port 3 of the housing 10 of switching valve 1 respectively with the first circuit port 4 and the second circuit port 5 of the housing 10 of the switching valve 1 to put in fluid communication the first access port A and the second access port B respectively with the blood withdrawal line 301 and the return line 302 of the extracorporeal blood circuit 300 so that to trigger the normal mode of operation of the extracorporeal blood circuit 300.

When the normal mode of operation is triggered by the selective connection body 6 of the switching valve 1, blood coming from the first access port A flows through the first transit port 2 of the housing 10 of the switching valve 1, a corresponding through channel 7a of the first pair 7a, 7b of channels of the selective connection body 6 of the switching valve 1, the first circuit port 4 of the housing 10 of the switching valve 1, the withdrawal line 301 of the extracorporeal blood circuit 300, the treatment unit 200, the blood return line 302 of the extracorporeal blood circuit 300, the second circuit port 5 of the housing 10 of the switching valve 1, the other corresponding through channel 7b of the first pair 7a, 7b of channels of the selective connection body 6 of the switching valve 1, the second transit port 3 of the housing 10 of the switching valve 1, to arrive at the second access port B.

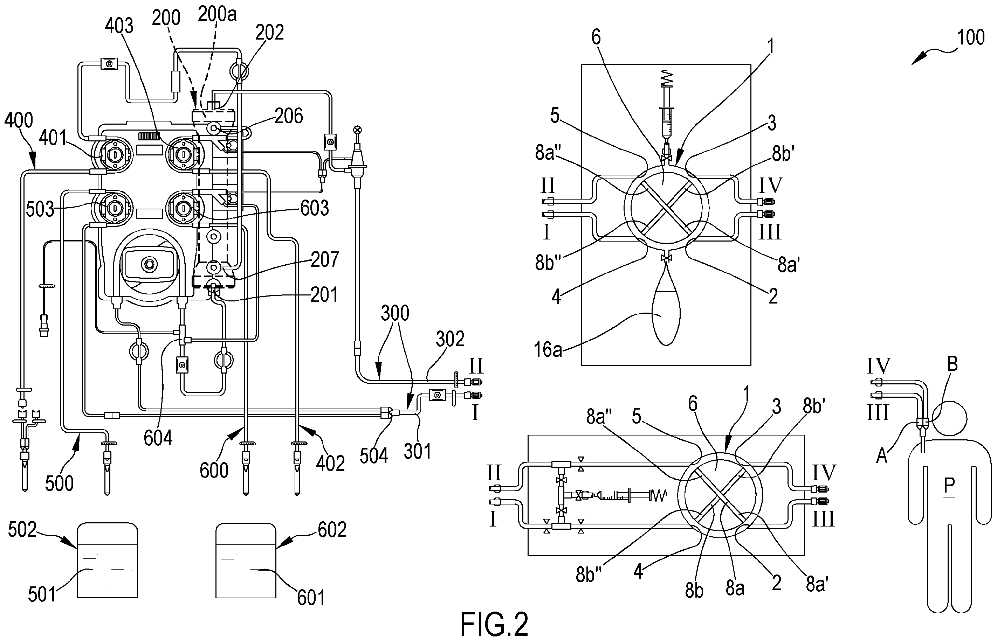

in the second switching condition (Figure 2), the first transit port 2 of the housing 10 of the switching valve 1 is fluidly connected to the second circuit port 5 of housing 10 of the switching valve 1 to put in fluid communication the first access port A with the blood return line 301 of the extracorporeal blood circuit 300, and the second transit port 3 of the housing 10 of the switching valve 1 is fluidly connected to the first circuit port 4 of the housing 10 of the switching valve 1 to put in fluid communication the second access port B with the withdrawal line 301 of the extracorporeal blood circuit 300. In particular, the second pair 8a, 8b of channels of the selective connection body 6 of the switching valve 1 fluidly connect the first transit port 2 and the second transit port 3 of the housing 10 of the switching valve 1 respectively with the second circuit port 5 and the first circuit port 4 of the housing 10 of the switching valve 1 to put in fluid communication the first access port A with the blood return line 302 of the extracorporeal blood circuit 300 and the second access port 302 with the blood withdrawal line 301 of the extracorporeal blood circuit 300 so that to trigger the reversing mode of operation of the extracorporeal blood circuit 300.

When the reversing mode of operation of the extracorporeal blood circuit 300 is triggered by the selective connection body 6, blood coming from the second access port B flows through the second transit port 3 of the housing 10 of the switching valve 1, the corresponding through channel 8b of the second pair 8a, 8b of channels of the selective connection body 6 of the switching valve 1, the first circuit port 4 of the housing 10 of the switching valve 1, the blood withdrawal line 301 of the extracorporeal blood circuit 300, the treatment unit 200, the blood return line 302 of the extracorporeal blood circuit 300, the second circuit port 5 of the housing 10 of the switching valve 1, the other corresponding through channel 8a of the second pair 8a, 8b of channels of the selective connection body 6 of the switching valve 1, the first transit port 2 of the housing 10 of the switching valve 1, to arrive at the first access port A.

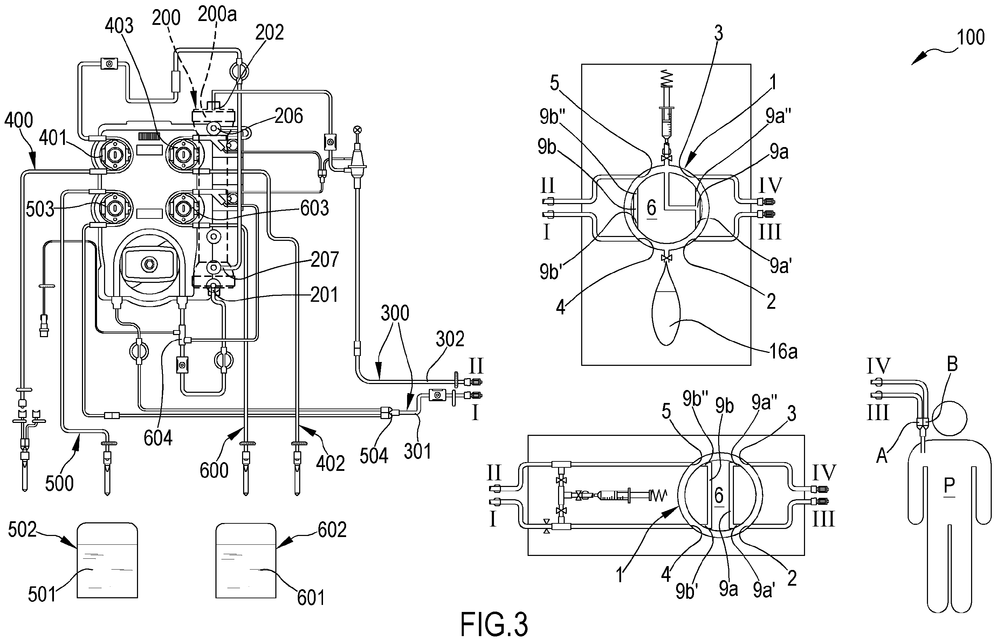

In the third switching condition (Figure 3), the first circuit port 4 and the second circuit port 5 of the housing 10 of the switching valve 1 are fluidly connected to each other to put in fluid communication the blood withdrawal line 301 with the blood return line 302 of the extracorporeal blood circuit 300, and the first transit port 2 and the second transit port 3 of housing 10 of the switching valve 1 are fluidly connected to each other to put in fluid communication the first access port A with the second access port B. In particular, the third pair 9a, 9b of through channels of the selective connection 6 of the switching valve 1 fluidly connect the first transit port 2 of the housing 10 of the switching valve 1 with the second transit port 3 of the housing of the switching valve 1, and the first circuit port 4 of the housing 10 of the switching valve 1 with the second circuit port 5 of the housing 10 of the switching valve 1, to respectively put in fluid communication the first access port A with the second access port B and the blood withdrawal line 301 with the blood return line 302 of the extracorporeal blood circuit 300 so that to trigger the recirculation mode of operation of the extracorporeal blood circuit 300.

When the recirculation mode of operation of the extracorporeal blood circuit 300 is triggered by the selective connection body 6 of the switching valve 1, blood coming from the first access port B, flows through the first transit port 2 of the housing 10 of the switching valve 1, the corresponding through channel 9a of the third pair 9a, 9b of channels of the selective connection body 6 of the switching valve 1, the second transit port 3 of the housing 10 of the switching valve 1 to arrive to the second access port B, and the blood inside the extracorporeal blood circuit 300 is circulated, according to a closed circuit, along the blood withdrawal line 301 of extracorporeal blood circuit 300, the treatment unit 200, the blood return line 302 of the extracorporeal blood circuit 300, the second circuit port 5 of the housing of the switching valve 1, the other corresponding through channel 9b of the third pair 9a, 9b of channels of the selective connection body 6 of the switching valve 1, the first circuit port 4 of the housing 10 of the switching valve 1, to arrive again at the blood withdrawal line 301 of the extracorporeal blood circuit 300 or according to an opposite circulation direction.

In the third condition, the selective connection body 6 of the switching valve 1 fluidly isolates the extracorporeal blood circuit 300 from the first A and the second access port B whereby these latter are not in fluid communication with the extracorporeal blood circuit 300.

When the through channels 7a, 7b, 8a, 8b, 9a, 9b need to be flushed it is possible to proceed according two different flushing operations.

The first flushing operation can be simultaneously performed during an extracorporeal blood treatment only on the through channels 7a, 7b, 8a, 8b, 9a, 9b of the selective connection body not connected to the ports 2, 3, 4, 5, of the housing 10 of the switching valve, namely the four through channels 7a, 7b, 8a, 8b, 9a, 9b (two pair of channels) in fluid communication with the interspace 14 defined between the housing 10 and the selective connection body 6. This type of flushing operation is normally carried out when the switching valve 1 is in the third switching condition, i.e. when the recirculation mode of operation is triggered by the selective connection body 6 of the switching valve 1.

The second flushing operation can be simultaneously carried out on all through channels 7a, 7b, 8a, 8b, 9a, 9b of the selective connection body 6 of the switching valve 1, but the extracorporeal blood treatment must be interrupted. In this situation, it is possible to put the selective connection body 6 according to a position wherein none of the through channels 7a, 7b, 8a, 8b, 9a, 9b are connected to the ports 2, 3, 4, 5 of the housing 10 so that each through channel 7a, 7b, 8a, 8b, 9a, 9b is in fluid communication with the interspace 14, being reachable by the flushing fluid arriving from the supply port 16.In the recirculation mode of operation, the transit ports 2, 3 of the housing 10 of the switching valve 1 and the tubes connecting such ports 2, 3 to the access ports A, B to the patient P can be flushed and cleared through the additional channel 19 which is provided on the corresponding through channel 9a of the third pair 9a, 9b of channels arranged on the patient side. In this situation, the additional channel 19 connects the interspace 14 with the transit ports 2, 3 by the corresponding through channel 9a of the third pair 9a, 9b of channels of the selective connection body 6. When the recirculation mode is switched, the discharge port 17 is closed and the flushing fluid is pushed through the corresponding trough channel 9a towards the transit ports 2, 3 of the housing 10 of the switching valve 1 until the tubes connected to them are cleared. In order to manage the flow of flushing fluid towards the access ports A, B, it could be necessary to provide clamps, valves and/or other similar devices.

The same can be applied when the extracorporeal blood treatment circuit 300 needs to be cleared. After interrupting the extracorporeal blood treatment, the discharge port 17 can be closed so that the flushing fluid is pushed into the lines 301, 302 of the extracorporeal blood circuit 300 through the additional channel 19 provided on the through channel 9b of the third pair 9a, 9b of channels arranged on the apparatus 100 side.

In the event that the through channel 9b of the third pair 9a, 9b of channels arranged on the apparatus side is provided with an additional channel 19, at least one clamp, a valve or any similar device is required to inhibit and isolate the additional channel 19 and the interspace 14 from saline supply and discharge, during the recirculation mode of operation.

### Control unit

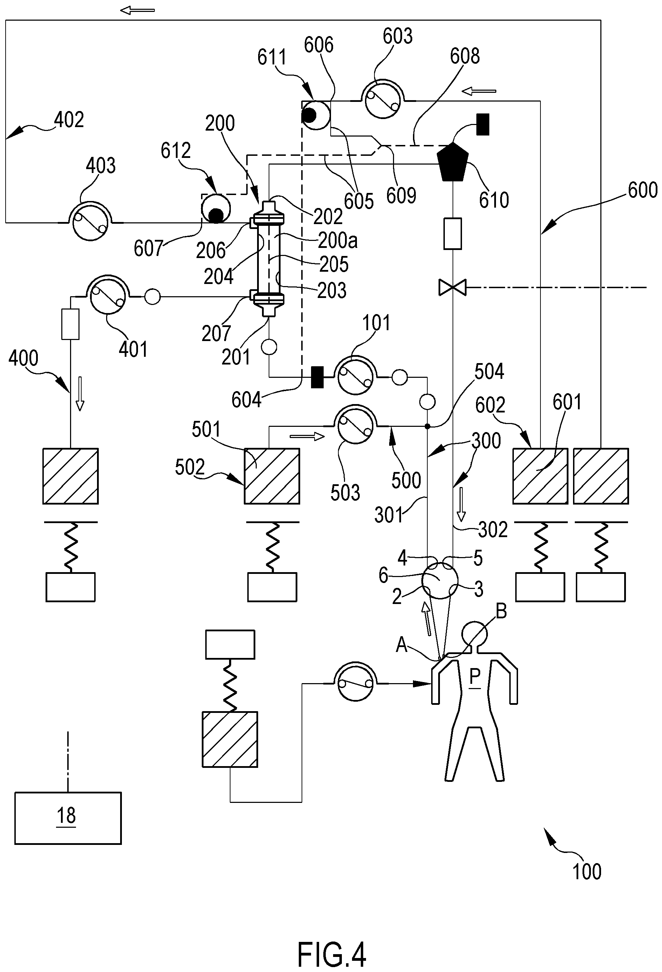

The extracorporeal blood treatment apparatus 100 and/or the extracorporeal blood circuit 300 are provided with a control unit 18 (schematically shown only in Figure 4) which is operatively connected to the switching valve 1 and is configured to rotationally drive the selective connection body 6 of the switching valve 1 between the first switching condition, the second switching condition and the third switching condition, to respectively trigger the normal mode of operation, the reversing mode of operation and the recirculation mode of the extracorporeal blood circuit 300.

In particular, the control unit 18 is connected to the actuator which is active on the transmission shaft 6d of the selective connection body 6 of the switching valve 1 to move, in particular to rotate, the transmission shaft 6d and, therefore, the selective connection body 6 inside the inner chamber 10a of the housing 10 of the switching valve 1 between the first switching condition (normal mode of operation), the second switching condition (reverse mode of operation) and the third switching condition (recirculation mode of operation).

The control unit 18 is also operatively connected to a flushing supply source to activate the supply of the flushing fluid, e.g. a saline infusion fluid, which flows from the flushing circuit through the supply port 16 of the housing 10 of the switching valve 1 to the interspace 14 defined between the housing 10 and the selective connection body 6 od the switching valve 1 so that to flush the through channels 7a, 7b, 8a, 8b, 9a, 9b of the selective connection body 6 not connected to the ports 2, 3, 4, 5, of the housing 10 and in fluid communication with the interspace 14. The activation of the supply of the flushing fluid can be executed during the operation of the blood treatment because the through channels 7a, 7b, 8a, 8b, 9a, 9b of the selective connection body 6 connected to the ports 2, 3, 4, 5, of the housing 10 are hermetically isolated from the interspace 14 by means of the corresponding seals 15.

The flushing supply source can be integrated into a circuit containing separate flushing capabilities. According to this embodiment, the switching valve can have a housing 10 not provided with an interspace, and inlet and a discharge port for a flushing fluid, and any additional channel. In that regard, the flushing sequence would then be governed by means of clamps/valves in order to emulates a traditional management manifold, known in the art.

Alternatively, the flushing supply source can be provided by a corresponding flushing fluid bag 16a, as shown in Figures 1, 2 and 3 (upper valve block), in fluid communication with the supply port 16 of the housing 10 of the switching valve 1.

When it is needed to provide the simultaneously flushing of all the through channels 7a, 7b, 8a, 8b, 9a, 9b of the selective connection body 6 of the switching valve 1, the extracorporeal blood treatment must be interrupted and the selective body 6 is driven by the control unit to reach a position inside the inner camber 10a of the housing 10 wherein none of the through channels 7a, 7b, 8a, 8b, 9a, 9b are connected to the ports 2, 3, 4, 5, of the housing 10, being all of them in fluid communication with the interspace 14. In such a situation, the activation of the flushing supply source, determines the simultaneously flushing of every through channel 7a, 7b, 8a, 8b, 9a, 9b of the selective connection body 6.

### Advantages

The extracorporeal blood treatment apparatus 100 described above and the switching valve 1 applied to such an extracorporeal blood treatment apparatus 100 allows to offer three mode of operations through the use of the same device, namely: the normal mode of operation wherein the blood coming from the patient flows through the treatment unit to return to the patient after being treated; the reverse mode of operation, wherein the blood wherein the blood coming from the patient flows through the treatment unit according to an opposite direction to return to the patient after being treated; the recirculation mode of operation wherein the blood contained into the extracorporeal blood circuit is circulated for a predetermined time period and the blood withdrawn from the patient is immediately redirect to the patient.

The three different modes of operation can be obtained by the altitude difference between the ports 2, 3, 4, 5, of the housing 10 of the switching valve 1 and the different altitude and configurations of the through channels 7a, 7b, 8a, 8b, 9a, 9b of the selective connection body 6 of the witching valve 1 which allow specific connections between different pairs of ports 2, 3, 4, 5, without mutual interferences. In fact, as shown in Figure 9, where the selective connection body 6 is shown from the above, it appears that two through channels 7a, 7b are substantially parallel for ensuring the normal mode of operation, two through channels 8a, 8b are crossed for ensuring the reverse mode of operation, and the other two through channel 9a, 9b features an internal angle into the core for ensuring the recirculation mode of operation.

The switching valve 1 described above minimize the areas of blood stagnation. In that regard, stagnation of blood di eliminated and/or mitigated by the option of flushing out the through channels 7a, 7b, 8a, 8b, 9a, 9b of the selective connection body 6 when unused. The flushing fluid can be a saline infusion fluid.

## Claims

1. An extracorporeal blood treatment apparatus (100) comprising:
a treatment unit (200);
an extracorporeal blood circuit (300) comprising a blood withdrawal line (301) connected to an inlet (201) of the treatment unit (200) and connectable to a first access port (A) or a second access port (B), opposite to the treatment unit (200), for the passage of blood inwards the extracorporeal blood circuit (300), and a blood return line (302) connected to an outlet (202) of the treatment unit (200) and connectable to the second access port (B) or the first access port (A), opposite to the treatment unit (200), for the passage of blood outwards the extracorporeal blood circuit (300);
at least one switching valve (1) in fluid communication with the blood withdrawal line (301) and the blood return line (302) of the extracorporeal blood circuit (300) and in fluid communication with the first access port (A) and the second access port (B), the switching valve (1) being switchable between:
(i) a first switching condition, wherein the first access port (A) and the second access port (B) are respectively fluidly connected to the blood withdrawal line (301) of the extracorporeal blood circuit (300) and the blood return line (302) of the extracorporeal blood circuit (300) to trigger a normal mode of operation of the extracorporeal blood circuit (300) wherein blood coming from the first access port (A) flows through the switching valve (1), the withdrawal line (301) of the extracorporeal blood circuit (300), the treatment unit (200), the blood return line (302) of the extracorporeal blood circuit (300), the switching valve (1) again, to arrive at the second access port (B);
(ii) a second switching condition, wherein the first access port (A) and the second access port (B) are respectively fluidly connected to the blood return line (302) of the extracorporeal blood circuit (300) and the blood withdrawal line (301) of the extracorporeal blood circuit (300) to trigger a reversing mode of operation of the extracorporeal blood circuit (300) wherein blood coming from the second access port (B) flows through the switching valve (1), the blood withdrawal line (301) of the extracorporeal blood circuit (300), the treatment unit (200), the blood return line (302) of the extracorporeal blood circuit (300), the switching valve (1) again, to arrive to the first access port (A);
(iii) a third switching condition, wherein the first access port (A) and the second access port (B) are fluidly connected to each other and the blood withdrawal line (301) and the blood return blood line (302) of the extracorporeal blood circuit (6) are fluidly connected to each other to trigger a recirculation mode of the extracorporeal blood circuit (300), wherein the extracorporeal blood circuit (300) is not in fluid communication with the first (A) and the second access port (B).

2. Switching valve (1) for an extracorporeal blood treatment apparatus (1) of the type comprising: a treatment unit (200) and an extracorporeal blood circuit (300) comprising a blood withdrawal line (301) connected to an inlet (201) of the treatment unit (200) and connectable to a first access port (A) or a second access port (B), opposite to the treatment unit (200), for the passage of blood inwards the extracorporeal blood circuit (300), and a blood return line (302) connected to an outlet (202) of the treatment unit (200) and connectable to the second access port (B) or the first access port (A), opposite to the treatment unit (200), for the passage of blood outwards the extracorporeal blood circuit (300),
the switching valve (1) being configured to be in fluid communication with the blood withdrawal line (301) and the blood return line (302) of the extracorporeal blood circuit (300) and in fluid communication with the first access port (A) and the second access port (B) and being switchable between:
(i) a first switching condition, wherein the first access port (A) and the second access port (B) are respectively fluidly connected to the blood withdrawal line (301) of the extracorporeal blood circuit (300) and the blood return line (302) of the extracorporeal blood circuit (300) to trigger a normal mode of operation of the extracorporeal blood circuit (300) wherein blood coming from the first access port (A) flows through the switching valve (1), the withdrawal line (301) of the extracorporeal blood circuit (300), the treatment unit (200), the blood return line (302) of the extracorporeal blood circuit (300), the switching valve (1) again, to arrive at the second access port (B);
(ii) a second switching condition, wherein the first access port (A) and the second access port (B) are respectively fluidly connected to the blood return line (302) of the extracorporeal blood circuit (300) and the blood withdrawal line (301) of the extracorporeal blood circuit (300) to trigger a reversing mode of operation of the extracorporeal blood circuit (300) wherein blood coming from the second access port (B) flows through the switching valve (1), the blood withdrawal line (301) of the extracorporeal blood circuit (300), the treatment unit (200), the blood return line (302) of the extracorporeal blood circuit (300), the switching valve (1) again, to arrive to the first access port (A);
(iii) a third switching condition, wherein the first access port (A) and the second access port (B) are fluidly connected to each other and the blood withdrawal line (301) and the blood return blood line (302) of the extracorporeal blood circuit (6) are fluidly connected to each other to trigger a recirculation mode of the extracorporeal blood circuit (300), wherein the extracorporeal blood circuit (300) is not in fluid communication with the first (A) and the second access port (B).

3. An extracorporeal blood treatment apparatus (100) or a switching valve (1) according to claim 1 or 2, wherein the switching valve (1) comprises a housing (10) defining an inner chamber (10a), the housing (10) being provided with: a first transit port (2) arranged to be in fluid communication with the first access port (A); a second transit port (3) arranged to be in fluid communication with the second access port (B); a first circuit port (4) arranged to be in fluid communication with the blood withdrawal line (301) of the extracorporeal blood circuit (300); and, a second circuit port (6) arranged to be in fluid communication with the blood return line (302) of the extracorporeal blood circuit (300), the switching valve (1) further comprising a selective connection body (6) configured to fluidly connect distinct pairs of ports (2, 3, 4, 5) of the housing (10) of the switching valve (1), optionally each port (2, 3, 4, 5) of the housing (10) of the switching valve (1) being provided with a tubular fitting (2a, 3a, 4a, 5a) protruding externally from the housing (10) of the switching valve (1), in particular the selective connection body (6) of the switching valve (1) rotatable occupying an inner chamber (10a) defined by housing (10) of the switching valve (1).

4. An extracorporeal blood treatment apparatus (100) or a switching valve (1) according to claim 3, wherein the selective connection body (6) comprises:
a first pair (7a, 7b) of through channels (7a, 7b, 8a, 8b, 9a, 9b) configured to respectively fluidly connect the first transit port (2) with the first circuit port (4) of the switching valve (1) and the second transit port (3) with the second circuit port (5) of the switching valve (1);
a second pair (8a, 8b) of through channels (7a, 7b, 8a, 8b, 9a, 9b), separate and distinct with respect to the through channels (7a, 7b, 8a, 8b, 9a, 9b) of the first pair (7a, 7b) and configured to respectively fluidly connect the first transit port (2) with the second circuit port (5) of the switching valve (1) and the second transit port (3) with the first circuit port (4) of the switching valve (1), and,
a third pair (9a, 9b) of through channels (7a, 7b, 8a, 8b, 9a, 9b) separate and distinct with respect to the through channels (7a, 7b, 8a, 8b, 9a, 9b) of the first (7a, 7b) and the second pairs (8a, 8b) and configured respectively to fluidly connect the first transit port (2) of the witching valve (1) to the second transit port (3) of the switching valve (1), and the first circuit port (4) of the switching valve (1) with the second circuit port (5) of the switching valve (1).

5. An extracorporeal blood treatment apparatus (100) or a switching valve (1) according to claim 4, wherein the selective connection body (6) is rotatable between:
(i) the first switching condition, wherein the first pair (7a, 7b) of through channels (7a, 7b, 8a, 8b, 9a, 9b) fluidly connects the first transit port (2) and the second transit port (3) of the switching valve (1) respectively with the first circuit port (4) and the second circuit port (5) of the switching valve (1) to put in fluid communication the first access port (A) and the second access port (B) respectively with the blood withdrawal line (301) and the return line (302) of the extracorporeal blood circuit (300), the first condition triggering a normal mode of operation of the extracorporeal blood circuit (300) wherein blood coming from the first access port (A), flows through the first transit port (2) of the switching valve (1), a corresponding through channel (7a) of the first pair (7a, 7b) of the selective connection body (6) of the switching valve (1), the first circuit port (4) of the switching valve (1), the withdrawal line (301) of the extracorporeal blood circuit (300), the treatment unit (200), the blood return line (302) of the extracorporeal blood circuit (300), the second circuit port (5) of the switching valve (1), the other corresponding through channel (7b) of the first pair (7a, 7b) of the selective connection body (6) of the switching valve (1), the second transit port (3) of the switching valve (1), to arrive at the second access port (B);
(ii) the second switching condition, wherein the second pair (8a, 8b) of through channels (7a, 7b, 8a, 8b, 9a, 9b) fluidly connect the first transit port (2) and the second transit port (3) of the switching valve (1) respectively with the second circuit port (5) and the first circuit port (4) of the switching valve (1) to put in fluid communication the first access port (A) with the blood return line (301) of the extracorporeal blood circuit (300) and the second access port (B) with the blood withdrawal line (301) of the extracorporeal blood circuit (300), the second condition triggering a reversing mode of operation of the extracorporeal blood circuit (300) wherein blood coming from the second access port (B) flows through the second transit port (3) of the switching valve (1), the corresponding through channel (8b) of the second pair (8a, 8b) of the selective connection body (6) of the switching valve (1), the first circuit port (4) of the switching valve (1), the blood withdrawal line (301) of the extracorporeal blood circuit (300), the treatment unit (200), the blood return line (302) of the extracorporeal blood circuit (300), the second circuit port (5) of the switching valve (1), the other corresponding through channel (8a) of the second pair (8a, 8b) of the selective connection body (6) of the switching valve (1), the first transit port (2) of the switching valve (1), to arrive at the first access port (A);
(iii) the third switching condition, wherein the third pair (9a, 9b) of through channels (7a, 7b, 8a, 8b, 9a, 9b) fluidly connect the first transit port (2) with the second transit port (3) of the switching valve (1), and the first circuit port (4) with the second circuit port (5) of the switching valve (1), to respectively put in fluid communication the first access port (A) with the second access port (B) and the blood withdrawal line (301) with the blood return line (302) of the extracorporeal blood circuit (300), the third condition triggering a recirculation mode of operation of the extracorporeal blood circuit (300) wherein blood coming from the first access port (A), flows through the first transit port (2) of the switching valve (1), the corresponding through channel (9a) of the third pair (9a, 9b) of the selective connection body (6) of the switching valve (1), the second transit port (3) of the switching valve (1) to arrive to the second access port (B), and blood inside the extracorporeal blood circuit (300) is able to be circulated, according to a closed circuit, along the blood withdrawal line (301) of extracorporeal blood circuit (300), the treatment unit (200), the blood return line (302) of the extracorporeal blood circuit (300), the second circuit port (5) of the switching valve (1), the other corresponding through channel (9b) of the third pair (9a, 9b) of the selective connection body (6) of the switching valve (1), the first circuit port (4) of the switching valve (1), to arrive again at the blood withdrawal line (301) of the extracorporeal blood circuit (300) or according to an opposite circulation direction, in the third condition the selective connection body (6) of the switching valve (1) fluidly isolates the extracorporeal blood circuit (300) from the first (A) and the second access port (B) whereby these latter are not in fluid communication with the extracorporeal blood circuit (300).

6. An extracorporeal blood treatment apparatus (100) or a switching valve (1) according to any one of the three previous claims, wherein the first and the second transit ports (2, 3) of the switching valve (1) are positioned at different levels or lying planes with respect to the levels or lying plains of the first and the second circuit ports (4, 5) of the switching valve (1), in particular each port (2, 3, 4, 5) of the switching valve (1) is positioned at a corresponding level or lying plane different with respect to the levels or lying planes of any one of the other ports (2, 3, 4, 5), optionally the first and the second transit ports (2, 3) of the switching valve (1) diverge from each other away from the housing (10) of the switching valve (1) and the first and the second circuit ports (4, 5) of the switching valve (1) diverge from each other away from the housing (10) of the switching valve (1), more optionally the tubular fittings (2a, 3a) corresponding to the first (2) and the second transit port (3) of the switching valve (1) diverge from each other, away from the housing (10) of the switching valve (1) and the tubular fittings (4a, 5a) corresponding to the first (4) and the second circuit port (5) of the switching valve (1) diverge from each other, away from the housing (10) of the housing (10) of the switching valve (1).

7. An extracorporeal blood treatment apparatus (100) or a switching valve (1) according to any one of the four previous claims, wherein the housing (10) of the switching valve 1 comprises:
a first wall (11), in particular substantially circular;
a second wall (12), in particular substantially cylindrical, transversally developing from the first wall (11); and,
a third wall (13), in particular substantially circular, transversally engaged to the second wall (12), on the opposite side to the first wall (11);
the first (2) and second transit port (3) of the switching valve (1) being positioned close to the third wall (13) of the housing (10) of the switching valve (1), while the first (4) and the second circuit port (5) of the switching valve (1) being positioned close to the first wall (11) of the housing (10) of the switching valve (1), optionally at least one of the first and second transit port (2, 3) of the switching valve (1) having a corresponding longitudinal development axis lying on a radial plane of the switching valve (1) on which a longitudinal development axis of at least one of the first and the second circuit port (4, 5) lies and each of the transit ports (2, 3) of the switching valve (1) has a corresponding longitudinal axis lying on a radial plane of the switching valve (1) on which a longitudinal development axis of a corresponding circuit port (4, 5) of the switching valve (1) lies.

8. An extracorporeal blood treatment apparatus (100) or a switching valve (1) according to claim 7, wherein the selective connection body (6) of the switching valve (1) comprises:
a first wall (6a), in particular substantially circular;
a second wall (6b), in particular substantially cylindrical, transversally developing from the first wall (6a), and;
a third wall (6c), in particular substantially circular transversally developing from the second wall (6b) and facing away from the first wall (6a);
the first wall (6a), the second wall (6b) and the third wall (6c) of the selective connection body (6) of the switching valve (1) respectively facing internally at the first wall (11), the second wall (12) and the third wall (13) of the housing (10) of the switching valve (1), optionally the selective connection body (6) of the switching valve (1) being provided with at least one transmission shaft (6d) engageable, directly or indirectly, to an actuator device able to move the transmission shaft (6d) and the selective connection body (6) of the switching valve (1), in particular the transmission shaft (6d) protruding from the third wall (6c) of the selective connection body (6) of the switching valve (1) and developing along a longitudinal axis (X) of the selective connection body (6) of the switching valve (1), through a corresponding through opening (10b) cut out in the center of the third wall (13).

9. An extracorporeal blood treatment apparatus (100) or a switching valve (1) according to claim 8, wherein each through channel (7a, 7b, 8a, 8b, 9a, 9b) of the selective connection body (6) of the switching valve (1) comprises two fluidic connection ends (7a', 7a", 7b', 7b", 8a', 8a", 8b', 8b", 9a', 9a", 9b', 9b") localized in correspondence of the second wall (6b) to provide a fluid communication with a corresponding pair of ports (2, 3, 4, 5) of the housing (10), one fluidic connection end (7a', 7b', 8a', 8b', 9a', 9b') defining an inlet for a fluid, while the other fluidic connection end (7a", 7b", 8a", 8b", 9a", 9b") defining an outlet for the same fluid.

10. An extracorporeal blood treatment apparatus (100) or a switching valve (1) according to claim 4 or any one of the five previous claims depending on claim 4, wherein:
the through channels of the first pair (7a, 7b) of channels of the selective connection body (6) develop alongside each other, without intersecting each other, a radial median plane (M) of the selective connection body (6) of the switching valve (1) being interposed between the through channels of the first pair (7a, 7b) of channels of the selective connection body (6), in particular the through channels of the first pair (7a, 7b) channels of the selective connection body (6) having a substantially rectilinear development, more in particular the through channels of the first pair (7a, 7b) of the selective connection body (6) having a development inclined with respect to the walls (6a, 6b, 6c) of the selective connection body (6);
the through channels of the second pair (8a, 8b) of channels of the selective connection body (6) cross a same radial median plane (M) of the latter, in particular the through channels of the second pair (8a, 8b) of channels of the selective connection body (6) having a substantially rectilinear development, more in particular the through channels of the second pair (8a, 8b) of channels of the selective connection body (6) having corresponding developments inclined with respect to the walls (6a, 6b, 6c) of the selective connection body (6) of the switching valve (1), optionally the through channels of the second pair (8a, 8b) of channels of the selective connection body (6) developing at different levels of the latter according to respective crossing directions;
the through channels of the third pair (9a, 9b) of channels of the selective body (6) of the switching valve (1) develop alongside each other, without intersecting, a same radial median plane (M) of the selective connection body (6) of the switching valve (1) being interposed between the through channels of the third pair (9a, 9b) of channels of the selective connection body (6), in particular each through channel of the third pair (9a, 9b) channels of the selective connection body (6) developing according to broken line, optionally having an elbow course, more in particular each through channel of the third pair (9a, 9b) of channels of the selective connection body (6) has a course having at least one bend (9a'", 9b‴) which defines a shape convex towards the other through channel of the third pair (9a, 9b) of channels of the selective connection body (6), and concave on the opposite side, optionally the through channels of the third pair (9a, 9b) of channels of the selective connection body (6) of the switching valve (1) have a development inclined with respect to the first wall (6a) and the third wall (6b) of the selective connection body (6), more optionally the through channels of the third pair (9a, 9b) of channels of the selective connection body (6) of the switching valve (1) developing at different levels of the latter.

11. An extracorporeal blood treatment apparatus (100) or a switching valve (1) according to claim 9 or 10 when depending on claim 9, wherein:
each fluidic connection end (8a', 8a", 8b', 8b") of each through channel of the second pair (8a, 8b) of channels of the selective connection body (6) of the switching valve (1) is localized along a same generatrix of the second wall (6b) of the selective connection body (6) where a corresponding fluidic end (7a', 7a", 7b', 7b") of a respective through channel of the first pair (7a, 7b) of the selective connection body (6) of the switching valve (1) is localized;
a fluidic connection end (9a', 9b') of each through channel of the third pair (9a, 9b) of channels of the selective connection body (6) of the switching valve (1) is localized along a same generatrix of the second wall (6b) of the selective connection body (6) where a fluidic connection end (7a', 7b') of one through channel of the first pair (7a, 7b) of channels of the selective connection body (6) of the switching valve (1) is localized and a fluidic connection end (8a', 8b') of one through channel of the second pair (8a, 8b) of the selective connection body (6) of the switching valve (1) is localized;
in particular, two generatrix of the second wall (6b) of the selective connection body (6), distinct and separate from each other, being provided with three fluidic connection ends (7a', 7b', 8a', 8b', 9a', 9b'), each corresponding to a specific through channel of the first (7a, 7b), the second (8a, 8b) and the third pair (9a, 9b) of channels of the selective connection body (6) respectively and other two generatrix of the second wall (6b) of the selective connection body (6), distinct and separate from each other and from any other generatrix, being provided with two fluidic connection ends (7a", 7b", 8a", 8b"), each corresponding to a specific through channel of the first (7a, 7b) and the second pair (8a, 8b) of channels of the selective connection body (6) respectively, further two generatrix of the second wall (6b) of the selective connection body (6), distinct and separate from each other and from any other generatrix, are provided with only one fluidic connection end (9a", 9b") of a corresponding through channel of the third pair (9a, 9b) of the selective connection body (6) of the switching valve (1).

12. An extracorporeal blood treatment apparatus (100) or a switching valve (1) according to claim 3 or any one of the eight previous claims depending on claim 3, wherein the selective connection body (6) is smaller than the inner chamber (10a) of the housing (10) of the switching valve (1) to define an interspace (14) between the housing (10) and the selective connection body (6), the housing (10) of the switching valve (1) being provided with a supply port (16) and a discharge port (17) in fluid communication with the interspace (14) and in fluid communication with a flushing circuit of the extracorporeal blood circuit (100), a flushing fluid arriving from the supply port (16) of the housing (10) and filling the interspace (14) provides the flushing of the through channels (7a, 7b, 8a, 8b, 9a, 9b) of the selective connection body (6) not connected to the transit ports (2, 3) and the circuit ports (4, 5) of the housing (10) to be discharged from the interspace (14) through the discharge port (17), the through channels (7a, 7b, 8a, 8b, 9a, 9b) of the selective connection body (6) of the switching valve (1) connected to the ports (2, 3, 4, 5) of the housing (10) of the switching valve (1) being isolated from the interspace (14) by means of corresponding seals (15) interposed between the selective connection body (6) and the housing (10).

13. An extracorporeal blood treatment apparatus (100) or a switching valve (1) according to claim 12 when depends on claim 7 or any one of the previous claims depending on claim 7, the discharge port (17) and the supply port (16) of the housing (10) of the switching valve (1) are respectively localized in correspondence of the first wall (11) and the third wall (13) of such a housing (10).

14. An extracorporeal blood treatment apparatus (100) or a switching valve (1) according to any one of the previous claims, wherein a control unit (13) is operatively connected to the switching valve (1) and is configured to drive the switching valve (1) between the first switching condition (i), the second switching condition (ii) and the third switching condition (iii), to respectively trigger the normal mode of operation, the reversing mode of operation and the recirculation mode operation.

15. An extracorporeal blood treatment apparatus (100) or a switching valve (1) according to claim 14, when depending on claim 3 or any one of the previous claims depending on claim 3, wherein the control unit (13) is configured to rotationally drive the selective connection body (6) of the switching valve (1) inside the inner chamber (10a) of the housing (10) of the switching valve (1) between the first switching condition (i), the second switching condition (ii) and the third switching condition (iii), to respectively trigger the normal mode of operation, the reversing mode of operation and the recirculation mode, in particular the control unit (13) being connected to the actuator to rotate the transmission shaft (6d) of the selective connection body (6) of the switching valve (1) and determine which through channels (7a, 7b, 8a, 8b, 9a, 9b) of the selective connection body (6) are connected to the ports (2, 3, 4, 5) of the housing (10) of the switching valve (1) and which through channels (7a, 7b, 8a, 8b, 9a, 9b) are not connected to the ports (2, 3, 4, 5) of the housing (10) of the switching valve (1).
